# EUROPEAN PATENT SPECIFICATION

(11) **EP 4 405 356 B1**
(45) Date of publication and mention of the grant of the patent: **18.11.2026**
(21) Application number: 22793720.8
(22) Date of filing: 23.09.2022
(51) Int. Cl.: C07D 471/14, A61P 15/00, A61P 25/00, A61P 25/08, A61P 25/16, A61P 25/18, A61P 25/22, A61P 25/24, A61P 25/28, A61P 29/00

(54) **PYRIDO DIAZEPINE DERIVATIVES AS GABA A GAMMA1 PAM**
PYRIDO-DIAZEPIN-DERIVATE ALS GABA-A-GAMMA1-PAM
DÉRIVÉS DE PYRIDO DIAZÉPINE EN TANT QUE PAM GAMMA1 GABA A

(30) Priority: 24.09.2021 EP 21198735
(43) Date of publication of application: 31.07.2024
(73) Proprietor: F. Hoffmann-La Roche AG, 4070 Basel (CH)
(72) Inventor: CECERE, Giuseppe, 4070 Basel (CH); GALLEY, Guido, 4070 Basel (CH); GOBBI, Luca, 4070 Basel (CH); HERNANDEZ, Maria-Clemencia, 4070 Basel (CH); KOBLET, Andreas, 4070 Basel (CH); OLIVARES MORALES, Andrés Miguel, 4070 Basel (CH); RUNTZ-SCHMITT, Valerie, 4070 Basel (CH)
(74) Representative: Graham Watt & Co
(86) International application number: PCT/EP2022/076467
(87) International publication number: WO 2023/046877

(56) References cited:
- WO-A2-03/082832
- GLORIA S. FORKUO ET AL: "Alleviation of Multiple Asthmatic Pathologic Features with Orally Available and Subtype Selective GABA A Receptor Modulators", MOLECULAR PHARMACEUTICS, vol. 14, no. 6, 5 June 2017 (2017-06-05), US, pages 2088 - 2098, XP055407375, ISSN: 1543-8384, DOI: 10.1021/acs.molpharmaceut.7b00183

## Description

### Field of the Invention

The present invention relates to organic compounds useful for therapy or prophylaxis in a mammal, and in particular to new pyrido diazepine derivatives that exhibit activity as GABAA γ1 receptor positive allosteric modulators (PAMs) and are thus useful for the treatment or prophylaxis of GABAA γ1 receptor related diseases or conditions.

### Background of the Invention

Receptors for the major inhibitory neurotransmitter, gamma-aminobutyric acid (GABA), are divided into two main classes: (1) GABA_{A} receptors, which are members of the ligand-gated ion channel superfamily and (2) GABA_{B} receptors, which are members of the G-protein linked receptor family. The GABA_{A} receptor complex which is a membrane-bound heteropentameric protein polymer is composed principally of α, β and γ subunits. GABA_{A} receptors are ligand-gated chloride channels and the principal mediators of inhibitory neurotransmission in the human brain.

There are 19 genes encoding for GABA_{A} receptor subunits that assemble as pentamers with the most common stoichiometry being two α, two β and one γ subunit. GABA_{A} subunit combinations give rise to functional, circuit, and behavioral specificity. GABA_{A} receptors containing the γ1 subunit (GABA_{A} γ1) are of particular interest due to their enriched expression in the limbic system and unique physiological and pharmacological properties. The GABA_{A} γ1 subunit-containing receptors, while less abundant (around 5-10 % of total expression of GABA_{A} receptors in the brain) than γ2 subunit-containing receptors exhibit an enriched brain mRNA and protein distribution in key brain areas such as extended amygdala (central, medial, and bed nucleus of the stria terminalis), lateral septum, hypothalamus, and pallidum/nigra. These structures form the interconnected core of a subcortical limbic circuit regulating motivated social and affective behaviors. In abnormal or disease conditions, hyper-recruitment of this circuit promotes anxiety, arousal, aggression, fear and defense while inhibiting foraging and social interactions.

Hyperactivity in limbic cortical regions (known to form a coordinated functional network with extended amygdala/ hypothalamus regions) which are key areas for processing of social and emotionally relevant stimuli, is the common hallmark of a variety of psychiatric, neurological, neurodevelopmental, neurodegenerative, mood, motivational and metabolic disorders. In such a disease state, and given the characteristic anatomical distribution of the γ1 subunit-containing GABA_{A} receptors, a GABA_{A} γ1 positive allosteric modulator (PAM) may be an effective treatment as a symptomatic or disease-modifying agent.

Multiple lines of evidence suggest that an imbalance between excitatory/inhibitory (E/I) neurotransmission arising from dysfunction of GABAergic signaling system, the main inhibitory neurotransmitter system in the brain, to be at the core of the pathogenesis a variety of CNS disorders. Given the distribution and function of GABA_{A} γ1 subunit-containing receptors in the CNS, they are very attractive targets for restoring levels of inhibition within key brain circuits and consequently the E/I balance in these conditions.

A CNS disorders of particular interest in the context of the present invention is autism spectrum disorder (ASD), including its core symptoms and associated comorbidities, such as anxiety and irritability, social anxiety disorder (social phobia) and generalized anxiety disorder. ASD is a complex, heterogeneous neurodevelopmental disorder characterized by impairments in two core domains: impairments in social interaction and communication, and presence of repetitive or restricted behaviors, interests, or activities (American Psychiatric Association 2013).

No approved pharmacological treatment exists for core symptoms of social deficits and restricted/repetitive behaviour of ASD, while only inadequate therapeutic options are available for most of ASD's affective and physiological co-morbidities. As a result, this disorder continues to be an area of high unmet medical need. Current approved treatments for associated symptoms of ASD are limited to the antipsychotics (Risperidone and Aripiprazole) indicated for the treatment of irritability associated with ASD symptoms. Emerging evidence suggests that the GABAergic system, the main inhibitory neurotransmitter system in the brain, plays a key role in the pathophysiology of ASD.

Both genetic and imaging studies using positron emission tomography study (PET) and magnetic resonance spectroscopy (MRS) suggest alterations in GABAergic signaling in ASD. The gene encoding GABA_{A} γ1, GABRG1, is located on chromosome 4 (mouse Chr.5) in a cluster with genes encoding α2, α4 and β1 GABA_{A} receptor subunits. Rare CNVs, including inversion of chromosome 4p12 disrupting GABRG1 have been observed in autistic siblings (Horike et al., 2006), as well as GABRG1 loss in one case of ADHD. Mutations in 4p12 gene cluster have been linked to increased risk of anxiety, substance abuse and eating disorders - providing a link between GABRG1/4p12 and affective dysfunction. MRS studies found altered GABA levels in ASD and in particular some recent studies showed reduced GABA and altered somatosensory function in children with ASD. In line with these observations, a reduced number of inhibitory interneurons were found from postmortem tissues of ASD and TS patients. Furthermore, reduced GABA synthesizing enzymes, glutamic acid decarboxylase (GAD) 65 and 67 were found in parietal and cerebellar cortices of patients with autism. Strong evidence in humans points to specific dysfunction in ASD of the limbic cortical regions known to form a coordinated functional network with GABA_{A} γ1 subunit-containing extended amygdala/ hypothalamus regions. These areas: Cortical/lateral amygdala, Insula, PFC, and Cingulate are recognized key for processing of social and emotionally relevant stimuli. While subcortical subnuclei that form specific partnerships with these areas, coordinating behavioural outcomes, are often difficult to study due to spatial resolution limitations, many lines of evidence point to hyper-recruitment of these cortical- to sub cortical connections in ASD. Moreover, recent high resolution studies provide a clear link between extended amygdala activity /functional connectivity and emotional state. Targeting such highly specified limbic subcortical regions, which exhibit substantial molecular and cellular diversity compared to the neocortex, will create a precision entry point for safe and specific therapeutic modulation of ASD-affected socio-affective circuits, while avoiding broad modulation of global brain state. Enhancement of GABA_{A} receptor activity by non-selective BZDs have been shown to ameliorate behavioral deficits in mouse models of ASD, however very narrow therapeutic margins were observed due to sedation mediated by the GABA_{A} α1γ2 subtype. These findings support the notion that rebalancing of GABAergic transmission *via* GABA_{A} γ1 receptors can improve symptoms in ASD without the side effects of non-selective benzodiazepines.

Compounds of the present invention are selective GABA_{A} γ1 receptor positive allosteric modulators (PAMs) that selectively enhance the function of γ1-containing GABA_{A} receptors by increasing GABAergic currents (influx of chloride) at a given concentration *(e.g.* EC₂₀) of gamma amino butyric acid (GABA). The compounds of the present invention have high PAM efficacy and binding selectivity for the γ1-containing subtypes (α5γ1, α2γ1, α1γ1) relative to the γ2-containing subtypes (e.g. α1γ2, α2γ2, α3γ2 and α5γ2). As such, compounds of the present invention are strongly differentiated from classical benzodiazepine drugs such as Alprazolam, Triazolam, Estazolam, and Midazolam, which are selective for the γ2-containing GABA_{A} subtypes and possess low affinity for the γ1-containing subtypes. Compatible with the γ1-subtypes brain distribution, selective GABA_{A} γ1 PAMs will restore GABAergic signaling in key brain regions (e.g. extended amygdala: central, medial, and bed nucleus of the stria terminalis, lateral septum, hypothalamus, and pallidum/nigra) without the side-effects of non-selective GABA_{A} modulators (e.g. benzodiazepines).

In view of the above, the selective GABA_{A} γ1 PAMs described herein and their pharmaceutically acceptable salts and esters are useful, alone or in combination with other drugs, as disease-modifying or as symptomatic agents for the treatment or prevention of acute neurological disorders, chronic neurological disorders and/or cognitive disorders, including autism spectrum disorders (ASD), Angelman syndrome, age-related cognitive decline, Rett syndrome, Prader-Willi syndrome, amyotrophic lateral sclerosis (ALS), fragile-X disorder, negative and/or cognitive symptoms associated with schizophrenia, tardive dyskinesia, anxiety, social anxiety disorder (social phobia), panic disorder, agoraphobia, generalized anxiety disorder, disruptive, impulse-control and conduct disorders, Tourette's syndrome (TS), obsessive-compulsive disorder (OCD), acute stress disorder, post-traumatic stress disorder (PTSD), attention deficit hyperactivity disorder (ADHD), sleep disorders, Parkinson's disease (PD), Huntington's chorea, Alzheimer's disease (AD), mild cognitive impairment (MCI), dementia, behavioral and psychological symptoms (BPS) in neurodegenerative conditions, multi-infarct dementia, agitation, psychosis, substance-induced psychotic disorder, aggression, eating disorders, depression, chronic apathy, anhedonia, chronic fatigue, seasonal affective disorder, postpartum depression, drowsiness, sexual dysfunction, bipolar disorders, epilepsy and pain.

WO03/082832 and G. S. Forkuo et al., Molecular Pharmaceutics, 14 (6), 2017, pages 2088-2098 disclose GABA_{A} receptor modulators.

### Summary of the Invention

In a first aspect, the present invention provides a compound of formula (I) 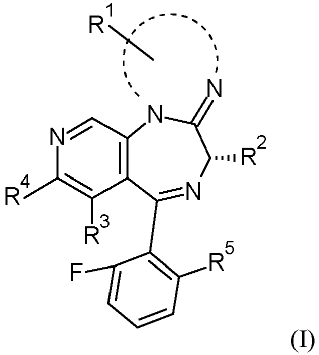 or a pharmaceutically acceptable salt thereof, wherein the variables are as defined herein.

In one aspect, the present invention provides a process of manufacturing the compounds of formula (I) described herein, wherein said process is as described in any one of Schemes 1 to 11 herein.

In a further aspect, the present invention provides a compound of formula (I) as described herein, when manufactured according to the processes described herein.

In a further aspect, the present invention provides a compound of formula (I) as described herein, or a pharmaceutically acceptable salt thereof, for use as therapeutically active substance.

In a further aspect, the present invention provides a pharmaceutical composition comprising a compound of formula (I) as described herein, or a pharmaceutically acceptable salt thereof, and a therapeutically inert carrier.

In a further aspect, the present invention provides a compound of formula (I) as described herein, or a pharmaceutically acceptable salt thereof, for use in a method for treating or preventing acute neurological disorders, chronic neurological disorders and/or cognitive disorders in a subject.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "alkyl" refers to a mono- or multivalent, e.g., a mono- or bivalent, linear or branched saturated hydrocarbon group of 1 to 6 carbon atoms ("C₁-C₆-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. Particularly preferred, yet non-limiting examples of alkyl include methyl and ethyl.

The term "alkoxy" refers to an alkyl group, as previously defined, attached to the parent molecular moiety via an oxygen atom. Unless otherwise specified, the alkoxy group contains 1 to 6 carbon atoms ("C₁-C₆-alkoxy"). In some preferred embodiments, the alkoxy group contains contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "cycloalkyl" as used herein refers to a saturated or partly unsaturated monocyclic or bicyclic hydrocarbon group of 3 to 10 ring carbon atoms ("C₃-C₁₀-cycloalkyl"). In some preferred embodiments, the cycloalkyl group is a saturated monocyclic hydrocarbon group of 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl, cycloheptyl, cyclopropenyl, cyclobutenyl, cyclopentenyl, cyclohexenyl, cycloheptenyl, and spiro[2.3]hexan-5-yl. Some preferred, yet non-limiting examples of cycloalkyl include cyclopropyl, cyclobutyl and cyclopentenyl.

The term "heterocyclyl" or "heterocycloalkyl" refers to a saturated or partly unsaturated mono- or bicyclic, preferably monocyclic ring system of 3 to 14 ring atoms, preferably 3 to 10 ring atoms, more preferably 3 to 8 ring atoms wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of heterocyclyl groups include azetidin-3-yl; azetidin-2-yl; oxetan-3-yl; oxetan-2-yl; piperidyl; piperazinyl; pyrrolidinyl; 2-oxopyrrolidin-1-yl; 2-oxopyrrolidin-3-yl; 5-oxopyrrolidin-2-yl; 5-oxopyrrolidin-3-yl; 2-oxo-1-piperidyl; 2-oxo-3-piperidyl; 2-oxo-4-piperidyl; 6-oxo-2-piperidyl; 6-oxo-3-piperidyl; 1-piperidinyl; 2-piperidinyl; 3-piperidinyl; 4-piperidinyl; morpholino (e.g., morpholin-2-yl or morpholin-3-yl); thiomorpholino, pyrrolidinyl (e.g., pyrrolidin-3-yl); 3-azabicyclo[3.1.0]hexan-6-yl; 2,5-diazabicyclo[2.2.1]heptan-2-yl; 2-azaspiro[3.3]heptan-2-yl; 2,6-diazaspiro[3.3]heptan-2-yl; and 2,3,3a,4,6,6a-hexahydro-1H-pyrrolo[3,4-c]pyrrol-5-yl. Some preferred, yet non-limiting examples of heterocyclyl are azetidinyl, oxetanyl, pyrrolidinyl, and thiomorpholino.

The term "hydroxy" refers to an -OH group.

The term "oxo" refers to an oxygen atom that is bound to the parent moiety via a double bond (=O).

The term "carbonyl" refers to a C=O group.

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Non-limiting examples of haloalkyl are fluoromethyl, difluoromethyl, trifluoromethyl, trifluoroethyl, 2-fluoroethyl, and 2,2-difluoroethyl. A particularly preferred, yet non-limiting example of haloalkyl is trifluoromethyl.

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are hydroxymethyl, hydroxyethyl (e.g. 2-hydroxyethyl), hydroxypropyl (e.g., 2-hydroxypropyl), and 3-hydroxy-3-methyl-butyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as formic acid, acetic acid, trifluoroacetic acid, propionic acid, glycolic acid, pyruvic acid, oxalic acid, maleic acid, malonic acid, succinic acid, fumaric acid, tartaric acid, lactic acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochlorides, fumarates, formates, lactates (in particular derived from L-(+)-lactic acid), tartrates (in particular derived from L-(+)-tartaric acid) and trifluoroacetates.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" or "prevention" as used herein includes: preventing or delaying the appearance of clinical symptoms of the state, disorder or condition developing in a subject and especially a human that may be afflicted with or predisposed to the state, disorder or condition but does not yet experience or display clinical or subclinical symptoms of the state, disorder or condition.

The term "subject" as used herein includes both humans and non-humans and includes but is not limited to humans, non-human primates, canines, felines, murines, bovines, equines, and porcines. In a particularly preferred embodiment, the term "subject" refers to humans.

The abbreviation uM means microMolar and is equivalent to the symbol µM.

The abbreviation uL means microliter and is equivalent to the symbol µL.

The abbreviation ug means microgram and is equivalent to the symbol µg.

### Compounds of the Invention

In a first aspect, the present invention provides a compound of formula (I) 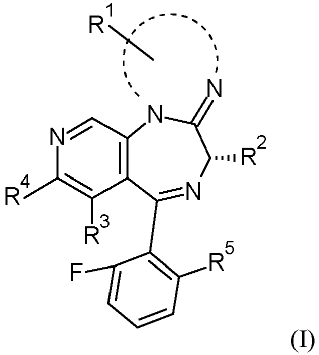 or a pharmaceutically acceptable salt thereof, wherein: 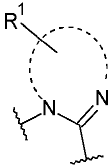 is selected from: 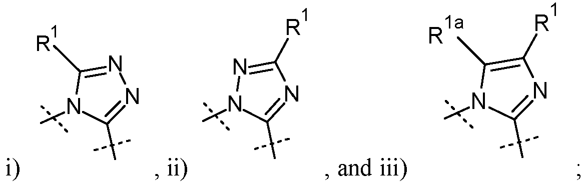
- R¹: is selected from hydrogen, C₁-C₆-alkyl, hydroxy-C₁-C₆-alkyl-NH-C(O)-, and a group ; and R^{1a} is hydrogen; or 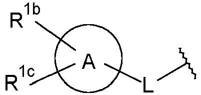
- R¹ and R^{1a},: taken together with the carbon atoms to which they are attached, form a C₃-C₁₀-cycloalkyl;
- R^{1b}: is selected from hydrogen, halogen, hydroxy, oxo, C₁-C₆-alkyl, and C₁-C₆-alkoxy;
- R^{1c}: is selected from hydrogen, hydroxy, and oxo;
- R²: is C₁-C₆-alkyl;
- R³: is chloro or bromo;
- R⁴: is selected from halogen, C₁-C₆-alkyl, halo-C₁-C₆-alkyl, and C₃-C₁₀-cycloalkyl;
- R⁵: is halogen;
- L: is selected from a covalent bond, carbonyl, -C(O)NH-,-NHC(O)-, and CH₂NHC(O)-; and
- A: is selected from 3-14-membered heterocycloalkyl and C₃-C₁₀-cycloalkyl.

In a preferred embodiment, the present invention provides a compound of formula (I) as described herein, or a pharmaceutically acceptable salt thereof, wherein 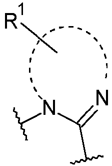 is selected from

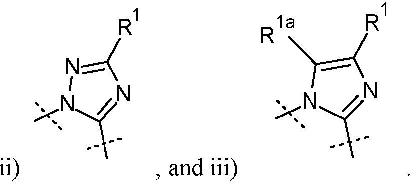

In a particularly preferred embodiment, the present invention provides a compound of formula (I) as described herein, or a pharmaceutically acceptable salt thereof, wherein 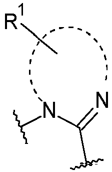 is

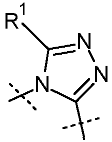

In a particularly preferred embodiment, the present invention provides a compound of formula (I) as described herein, or a pharmaceutically acceptable salt thereof, wherein 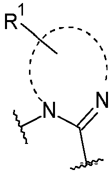 is

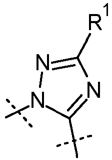

In a particularly preferred embodiment, the present invention provides a compound of formula (I) as described herein, or a pharmaceutically acceptable salt thereof, wherein 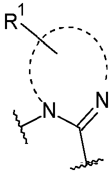 is

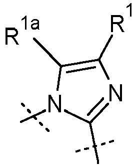

In one embodiment, the present invention provides a compound of formula (I) as described herein, or a pharmaceutically acceptable salt thereof, wherein
- R¹: is selected from C₁-C₆-alkyl, hydroxy-C₁-C₆-alkyl-NH-C(O)-, and a group 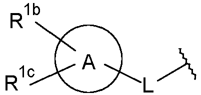 and R^{1a} is hydrogen; or
- R¹ and R^{1a},: taken together with the carbon atoms to which they are attached, form a C₃-C₁₀-cycloalkyl; and
- R^{1b}, R^{1c}, A, and L: are as defined herein.

In one embodiment, the present invention provides a compound of formula (I) as described herein, or a pharmaceutically acceptable salt thereof, wherein
- R¹: is selected from C₁-C₆-alkyl, hydroxy-C₁-C₆-alkyl-NH-C(O)-, and a group 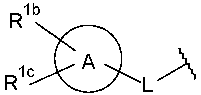 R^{1a} is hydrogen; and
- R^{1b}, R^{1c}, A, and L: are as defined in claim 1.

In one embodiment, the present invention provides a compound of formula (I) as described herein, or a pharmaceutically acceptable salt thereof, wherein
R¹ and R^{1a}, taken together with the carbon atoms to which they are attached, form a C₃-C₁₀-cycloalkyl; and
R^{1b}, R^{1c}, A, and L are as defined in claim 1.

In a preferred embodiment, the present invention provides a compound of formula (I) as described herein, or a pharmaceutically acceptable salt thereof, wherein
- R¹: is selected from C₁-C₆-alkyl, hydroxy-C₁-C₆-alkyl-NH-C(O)-, and a group 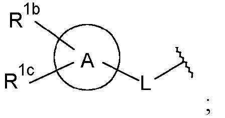
- R^{1b}: is C₁-C₆-alkyl;
- R^{1c}: is hydroxy;
- L: is carbonyl; and
- A: is a 3-14-membered heterocycle.

In a particularly preferred embodiment, the present invention provides a compound of formula (I) as described herein, or a pharmaceutically acceptable salt thereof, wherein
- R¹: is selected from 2-hydroxyethyl-NH-C(O)-, 2-hydroxypropyl-NH-C(O)-, methyl, and a group ; 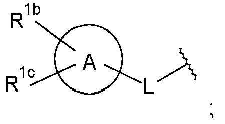
- R^{1b}: is methyl;
- R^{1c}: is hydroxy;
- L: is carbonyl; and
- A: is a azetidinyl.

In a preferred embodiment, the present invention provides a compound of formula (I) as described herein, or a pharmaceutically acceptable salt thereof, wherein R¹ is hydroxy-C₁-C₆-alkyl-NH-C(O)-.

In a preferred embodiment, the present invention provides a compound of formula (I) as described herein, or a pharmaceutically acceptable salt thereof, wherein R¹ is 2-hydroxyethyl-NH-C(O)-.

In a preferred embodiment, the present invention provides a compound of formula (I) as described herein, or a pharmaceutically acceptable salt thereof, wherein R² is methyl.

In a preferred embodiment, the present invention provides a compound of formula (I) as described herein, or a pharmaceutically acceptable salt thereof, wherein R³ is chloro.

In a preferred embodiment, the present invention provides a compound of formula (I) as described herein, or a pharmaceutically acceptable salt thereof, wherein R⁴ is halo-C₁-C₆-alkyl.

In a particularly preferred embodiment, the present invention provides a compound of formula (I) as described herein, or a pharmaceutically acceptable salt thereof, wherein R⁴ is CF₃.

In one embodiment, the present invention provides a compound of formula (I) as described herein, or a pharmaceutically acceptable salt thereof, wherein R⁵ is halogen.

In one embodiment, the present invention provides a compound of formula (I) as described herein, or a pharmaceutically acceptable salt thereof, wherein R⁵ is fluoro or chloro.

In a preferred embodiment, the present invention provides a compound of formula (I) as described herein, or a pharmaceutically acceptable salt thereof, wherein R⁵ is fluoro.

In a preferred embodiment, the present invention provides a compound of formula (I) as described herein, or a pharmaceutically acceptable salt thereof, wherein R⁵ is chloro.

In a preferred embodiment, the present invention provides a compound of formula (I) as described herein, or a pharmaceutically acceptable salt thereof, wherein:
- R¹: is selected from C₁-C₆-alkyl, hydroxy-C₁-C₆-alkyl-NH-C(O)-, and a group 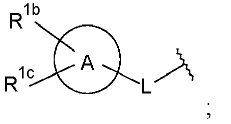
- R^{1b}: is C₁-C₆-alkyl;
- R^{1c}: is hydroxy;
- R²: is C₁-C₆-alkyl;
- R³: is chloro;
- R⁴: is halo-C₁-C₆-alkyl;
- R⁵: is halogen;
- L: is carbonyl; and
- A: is a 3-14-membered heterocycle.

In a particularly preferred embodiment, the present invention provides a compound of formula (I) as described herein, or a pharmaceutically acceptable salt thereof, wherein:
- R¹: is selected from methyl, 2-hydroxyethyl-NH-C(O)-, 2-hydroxypropyl-NH-C(O)-, and a group 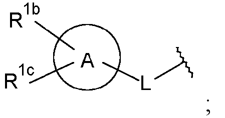
- R^{1b}: is methyl;
- R^{1c}: is hydroxy;
- R²: is methyl;
- R³: is chloro;
- R⁴: is CF₃;
- R⁵: is fluoro;
- L: is carbonyl; and
- A: is azetidinyl.

In one embodiment, the present invention provides a compound of formula (I) as described herein, or a pharmaceutically acceptable salt thereof, wherein said compound of formula (I) is selected from:
(7*S*)-11,12-dichloro-9-(2,6-difluorophenyl)-3,7-dimethyl-2,4,5,8,13-pentazatricyclo[8.4.0.02,6]tetradeca-1(10),3,5,8,11,13-hexaene;
(7*S*)-11-chloro-12-cyclopropyl-9-(2,6-difluorophenyl)-3,7-dimethyl-2,4,5,8,13-pentazatricyclo[8.4.0.02,6]tetradeca-1(10),3,5,8,11,13-hexaene;
(7*S*)-11-chloro-9-(2,6-difluorophenyl)-3,7,12-trimethyl-2,4,5,8,13-pentazatricyclo[8.4.0.02,6]tetradeca-1(10),3,5,8,11,13-hexaene;
(7*S*)-11-chloro-9-(2,6-difluorophenyl)-3,7-dimethyl-12-(trifluoromethyl)-2,4,5,8,13-pentazatricyclo[8.4.0.02,6]tetradeca-1(10),3,5,8,11,13-hexaene;
(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene;
azetidin-1-yl-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-fluoroazetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxyazetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-methoxyazetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(1,1-dioxo-1,4-thiazinan-4-yl)methanone;
*N*-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]oxetane-3-carboxamide;
1-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]pyrrolidin-2-one;
(7*S*)-11-chloro-9-(2,6-difluorophenyl)-*N*-[(2*S*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-(2-hydroxyethyl)-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-[(2*R*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2-chloro-6-fluoro-phenyl)-3,7-dimethyl-12-(trifluoromethyl)-2,4,5,8,13-pentazatricyclo[8.4.0.02,6]tetradeca-1(10),3,5,8,11,13-hexaene;
(7*S*)-11-chloro-9-(2,6-difluorophenyl)-4,7-dimethyl-12-(trifluoromethyl)-2,5,8,13-tetrazatricyclo[8.4.0.02,6]tetradeca-1(10),3,5,8,11,13-hexaene;
(7*S*)-11-chloro-9-(2,6-difluorophenyl)-*N*-(2-hydroxyethyl)-7-methyl-12-(trifluoromethyl)-2,5,8,13-tetrazatricyclo[8.4.0.02,6]tetradeca-1(10),3,5,8,11,13-hexaene-4-carboxamide;
(10*S*)-6-chloro-8-(2,6-difluorophenyl)-10-methyl-5-(trifluoromethyl)-1,4,9,12-tetrazatetracyclo[9.6.0.02,7.013,17]heptadeca-2(7),3,5,8,11,13(17)-hexaene;
(7*S*)-11-chloro-9-(2,6-difluorophenyl)-*N*-(2-hydroxy-2-methyl-propyl)-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-[(1-hydroxycyclopropyl)methyl]-7-methyl-12-(trifluoromethyl)-2,3,5,8,13-pentazatricyclo[8.4.0.02,6]tetradeca-1(10),3,5,8,11,13-hexaene-4-carboxamide;
(7S)-11-chloro-9-(2,6-difluorophenyl)-*N*-*cis*-(3-hydroxycyclobutyl)-7-methyl-12-(trifluoromethyl)-2,3,5,8,13-pentazatricyclo[8.4.0.02,6]tetradeca-1(10),3,5,8,11,13-hexaene-4-carboxamide; and
(7S)-11-chloro-9-(2,6-difluorophenyl)-*N*-*trans*-(3-hydroxycyclobutyl)-7-methyl-12-(trifluoromethyl)-2,3,5,8,13-pentazatricyclo[8.4.0.02,6]tetradeca-1(10),3,5,8,11,13-hexaene-4-carboxamide.

In preferred embodiment, the present invention provides a compound of formula (I) as described herein, or a pharmaceutically acceptable salt thereof, wherein said compound of formula (I) is selected from:
(7*S*)-11-chloro-9-(2,6-difluorophenyl)-3,7-dimethyl-12-(trifluoromethyl)-2,4,5,8,13-pentazatricyclo[8.4.0.02,6]tetradeca-1(10),3,5,8,11,13-hexaene;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanone;
(7*S*)-11-chloro-9-(2,6-difluorophenyl)-*N*-[(2*S*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-(2-hydroxyethyl)-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-[(2*R*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide; and
(7*S*)-11-chloro-9-(2,6-difluorophenyl)-*N*-(2-hydroxyethyl)-7-methyl-12-(trifluoromethyl)-2,5,8,13-tetrazatricyclo[8.4.0.02,6]tetradeca-1(10),3,5,8,11,13-hexaene-4-carboxamide.

In a particularly preferred embodiment, the present invention provides a compound of formula (I) as described herein, or a pharmaceutically acceptable salt thereof, wherein said compound of formula (I) is (7*S*)-11-chloro-9-(2,6-difluorophenyl)-*N*-(2-hydroxyethyl)-7-methyl-12-(trifluoromethyl)-2,3,5,8,13-pentazatricyclo[8.4.0.02,6]tetradeca-1(10),3,5,8,11,13-hexaene-4-carboxamide.

In a particularly preferred embodiment, the present invention provides a compound of formula (I) as described herein, or a pharmaceutically acceptable salt thereof, wherein said compound of formula (I) is (7*S*)-11-chloro-9-(2,6-difluorophenyl)-*N*-(2-hydroxyethyl)-7-methyl-12-(trifluoromethyl)-2,5,8,13-tetrazatricyclo[8.4.0.02,6]tetradeca-1(10),3,5,8,11,13-hexaene-4-carboxamide.

In particularly preferred embodiment, the present invention provides a compound of formula (I) as described herein, or a pharmaceutically acceptable salt thereof, wherein said compound of formula (I) is (7*S*)-11-chloro-9-(2,6-difluorophenyl)-3,7-dimethyl-12-(trifluoromethyl)-2,4,5,8,13-pentazatricyclo[8.4.0.02,6]tetradeca-1(10),3,5,8,11,13-hexaene.

In particularly preferred embodiment, the present invention provides a compound of formula (I) as described herein, or a pharmaceutically acceptable salt thereof, wherein said compound of formula (I) is [(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanone.

In particularly preferred embodiment, the present invention provides a compound of formula (I) as described herein, or a pharmaceutically acceptable salt thereof, wherein said compound of formula (I) is (7*S*)-11-chloro-9-(2,6-difluorophenyl)-*N*-[(2*S*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide.

In particularly preferred embodiment, the present invention provides a compound of formula (I) as described herein, or a pharmaceutically acceptable salt thereof, wherein said compound of formula (I) is (7*S*)-11-chloro-9-(2,6-difluorophenyl)-*N*-[(2*R*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide.

In one embodiment, the present invention provides pharmaceutically acceptable salts of the compounds of formula (I) as described herein, especially pharmaceutically acceptable salts selected from hydrochlorides, fumarates, lactates (in particular derived from L-(+)-lactic acid), tartrates (in particular derived from L-(+)-tartaric acid) and trifluoroacetates. In yet a further particular embodiment, the present invention provides compounds according to formula (I) as described herein (i.e., as "free bases" or "free acids", respectively).

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

Processes for the manufacture of the compound of formula (I) as described herein are also an object of the invention.

The preparation of compounds of formula (I) of the present invention may be carried out in sequential or convergent synthetic routes. Syntheses of the compounds of the invention are shown in the following schemes. The skills required for carrying out the reactions and purifications of the resulting products are known to those skilled in the art. The substituents and indices used in the following description of the processes have the significance given herein before and in the claims, unless indicated to the contrary. In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 3rd Edition, Richard C. Larock. John Wiley & Sons, New York, NY. 2018). It is convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux temperature. The time required for the reaction may also vary widely, depending on many factors, notably the reaction temperature and the nature of the reagents. However, a period of from 0.5 h to several days will usually suffice to yield the described intermediates and compounds. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The preparation of compounds of formula (I) of the present invention may be carried out in sequential or convergent synthetic routes. Syntheses of the invention are shown in the following general schemes. The skills required for carrying out the reactions and purifications of the resulting products are known to those skilled in the art. The substituents and indices used in the following description of the processes have the significance given herein before unless indicated to the contrary.

In more detail, the compounds of formula (I) can be manufactured by the methods given below, by the methods given in the examples or by analogous methods. Appropriate reaction conditions for the individual reaction steps are known to a person skilled in the art. The reaction sequence is not limited to the one displayed in schemes 1 - 11, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The present compounds of formula (Ia) and their pharmaceutically acceptable salts can be prepared by the process described in Scheme 1.

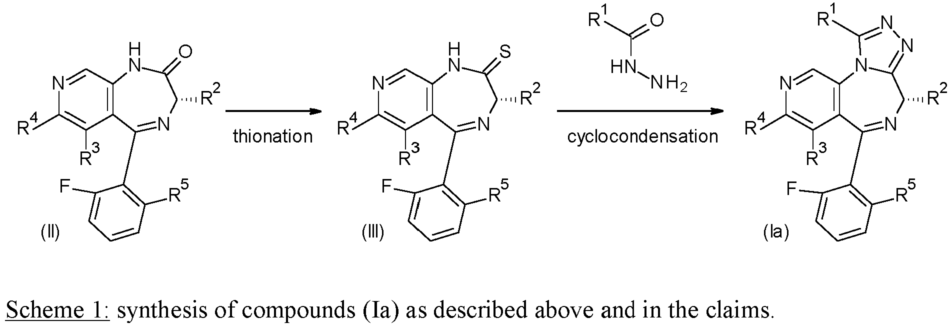

According to Scheme 1, a compound of formula (Ia) can be prepared in one or two steps starting from lactams of formula (II). Following thionation reaction using Lawesson's reagent or P₂S₅, lactams (II) are converted to corresponding thiolactams (III). Their reaction with hydrazides *via* a Pellizzari type process yields 1,2,4-triazoles of general formula (Ia). Alternatively, compounds (Ia) can be directly accessed by reaction of lactams (II) with an hydrazide using bis(2-oxo-3-oxazolidinyl)phosphinic chloride (BOP-Cl) in presence of a base (NaH) in tetrahydrofuran.

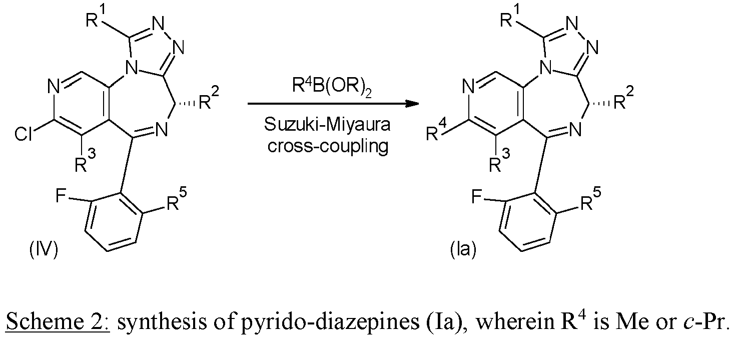

According to Scheme 2, pyrido-diazepines (Ia), wherein R⁴ is methyl or cyclopropyl, can be obtained by a palladium-catalyzed Suzuki-Miyaura cross-coupling reaction between 2-chloropyridines (IV) and boron reagents such as trimethylboroxine or cyclopropylboronic acid, using an inorganic base (e.g. K₂CO₃ or K₃PO₄) in 1,4-dioxane or toluene at elevated temperatures.

Triazoles of formula (Ib) can be prepared according to a process described in Scheme 3.

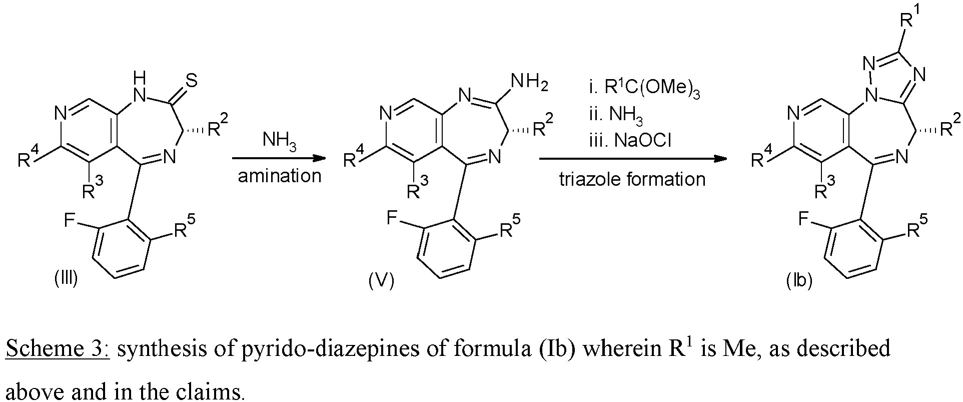

According to Scheme 3, 1,2,4-triazoles (Ib) can be prepared, starting from thiolactames (III), by treatment with ammonia in methanol to form amidines (V). Following their sequential reaction with triethyl or trymethyl orthoacetate, treatment with ammonia in methanol, and final ring closure by reaction with sodium hypochlorite in water and methanol, final derivatives (Ib) were obtained.

In a further embodiment of the invention, compounds of formula (Ib) wherein R¹ is an amide can be prepared according to a process described in Scheme 4.

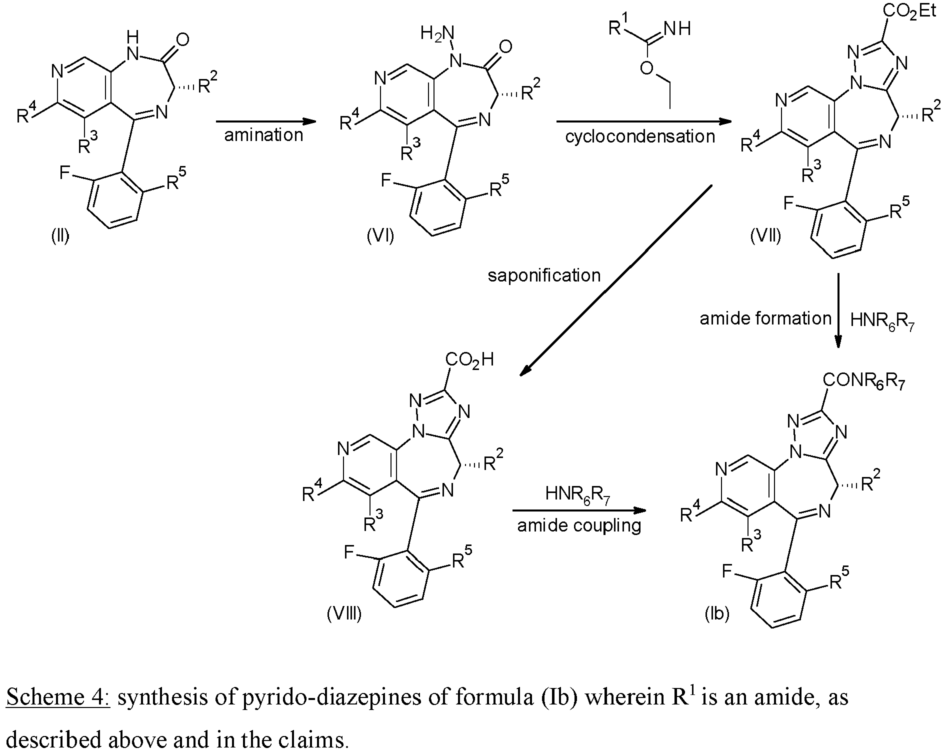

Electrophilic amination of lactams (II) using and O-(diphenylphosphinyl)hydroxylamine yields intermediates of formula (VI). Their thermal cyclocondensation reaction with imidates provides 1,2,4-triazoles (VII). Final derivatives of formula (Ib) can be obtained by either saponification of the ethyl ester (VII) to the carboxylic acid (VIII) under basic conditions (*e.g.* NaOH or LiBr, Et₃N), followed by standard amide coupling with amines HNR⁵R⁶ (*e.g.* HATU, DIPEA or PyBOP, DIPEA), or by direct reaction of the ester (VII) with amines HNR⁵R⁶ in ethanol.

Compounds of formula (Ib) wherein R¹ is a reversed amide can be prepared according to a process described in Scheme 5.

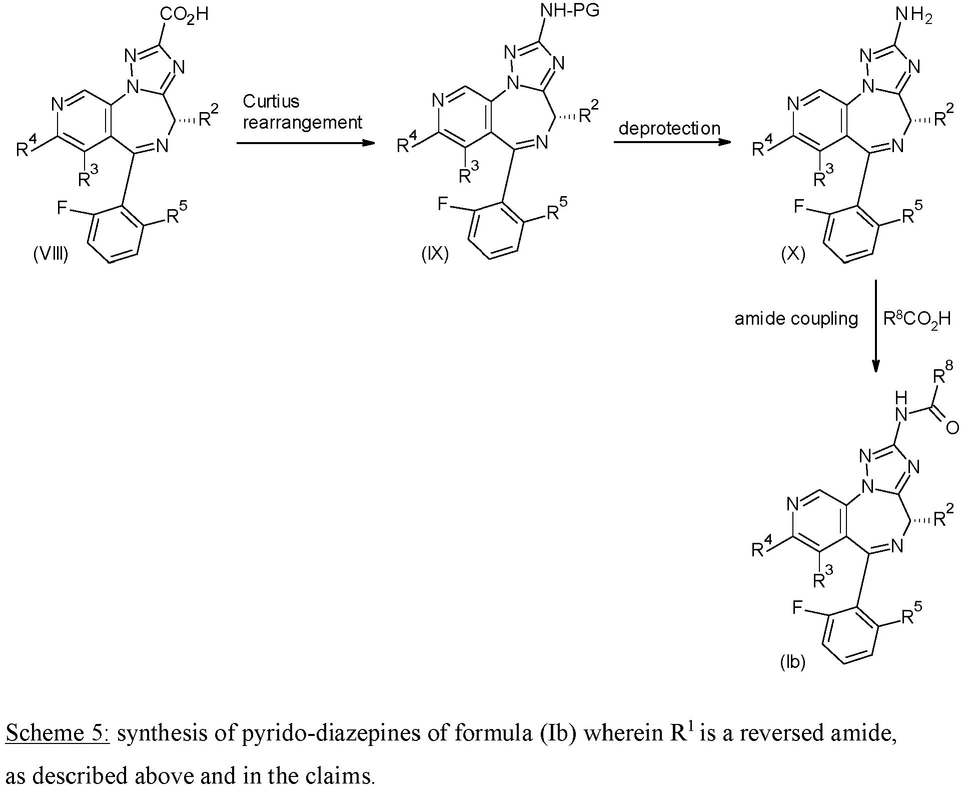

According to Scheme 5, *N*-protected protected triazoles (IX) can be accessed by a Curtius rearrangement when carboxylic acids (VIII) are heated with diphenylphosphoryl azide in presence of a base (*e.g.* Et₃N). Removal of *N*-Boc protecting group can be accomplished with mineral acids (*e.g.* HCl) or organic acids (*e.g.* trifluoroacetic acid) to yield amines of formula (X), which, in turn, can be coupled with a carboxylic acid R⁸CO₂H (*e.g.* POCl₃ in pyridine) to provide final derivatives (Ib).

Furthermore, according to Scheme 6, 4-chlorobutanamides (XI) can be cyclized in presence of a base (*e.g.* Et₃N) to form 5-membered lactams of formula (Ib).

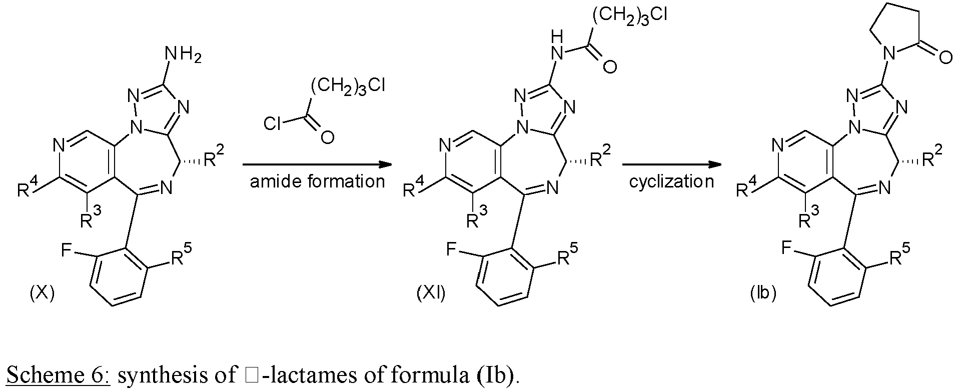

In a further embodiments of the invention, imidazoles of formula (Ic) can be prepared according to a process described in Scheme 7.

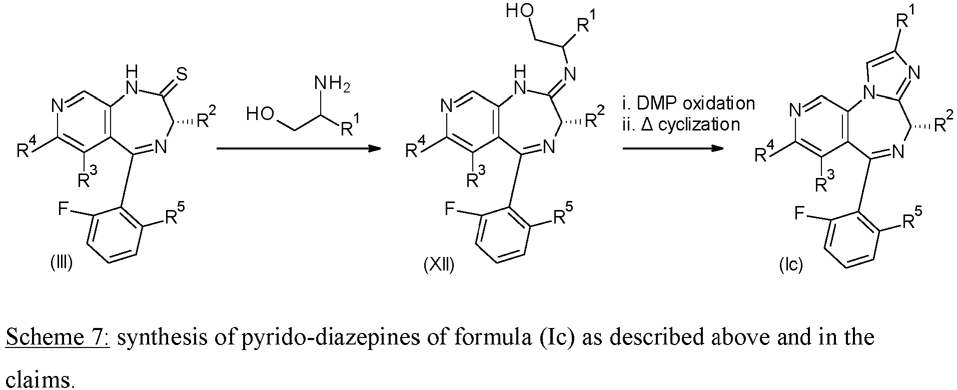

According to Scheme 7, thiolactams (III) can be reacted with an aminoalcohol of general formula HOCH₂CH(NH₂)R¹ to form substituted amidines (XII). Final compounds (Ic) are obtained in two steps synthesis by Dess-Martin oxidation of alcohols (XII) to corresponding aldehydes, followed by thermal cyclization.

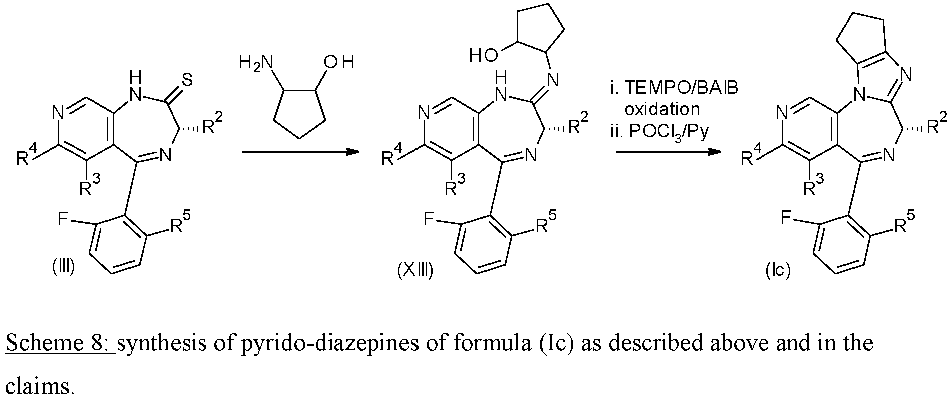

According to Scheme 8, in the case of 2-aminocyclopentanol, the alcohol (XII) can be oxidized with TEMPO and phenyl-λ3-iodanediyl diacetate (BAIB), followed by cyclization with POCl₃ and pyridine to provide imidazoles (Ic).

In alternative, imidazoles of general formula (Ic) can be prepared via ester intermediates (XV) as detailed in Scheme 9.

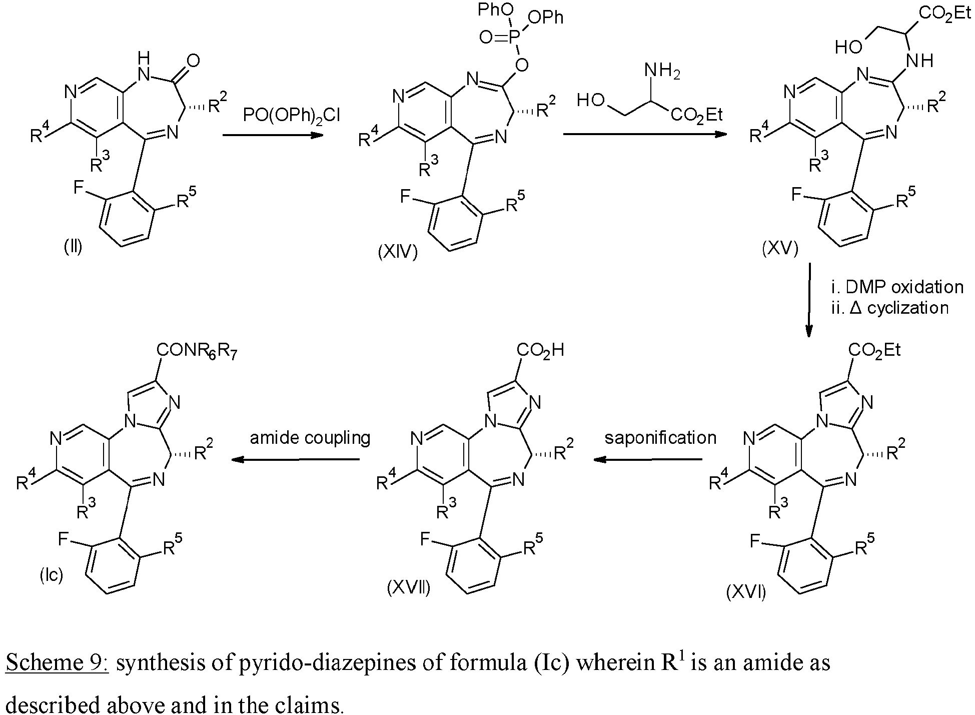

According to Scheme 9, lactams (II) can be activated by reaction with [chloro(phenoxy)phosphoryl]oxybenzene in presence of a base *(e.g.* NaH) to form a diphenyl phosphonate of general formula (XIV) which, in turn, can be reacted with aminoalcohols HOCH₂CH(NH₂)R¹ to form amidines (XV). Subsequent oxidation with Dess-Martin periodinane followed by thermal cyclization leads to ethyl esters (XVI). Finally, their saponification to carboxylic acids (XVII) can be performed with saturated aqueous lithium bromide in presence of a base (*e.g.* Et₃N) and their amide coupling with HATU, DIPEA provides the desired imidazoles of formula (Ic).

The synthesis of lactams (II) is highlighted in Scheme 10.

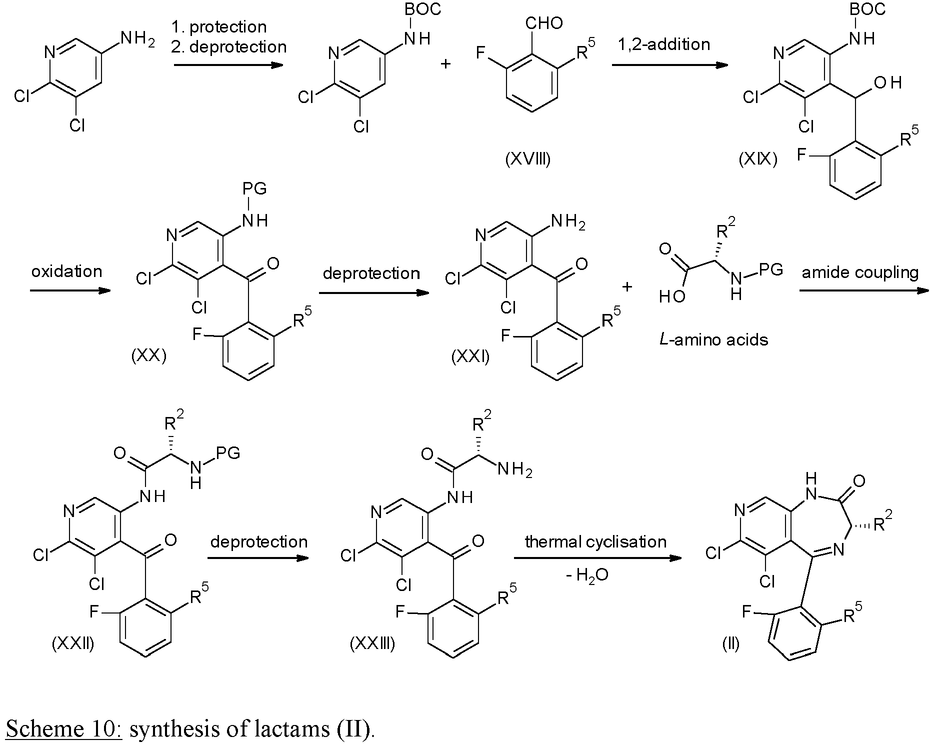

Commercially available 5,6-dichloropyridin-3-amine can be protected with a suitable protecting group such as *tert*-butyloxycarbonyl by treatment with di-*tert*-butyl dicarbonate in presence of a base (e.g. diisopropylethylamine), followed by treatment with trifluoroacetic acid in dichloromethane to provide *tert*-butyl *N*-(5,6-dichloro-3-pyridyl)carbamate. Regioselective organolithium formation by a metalation reaction at low temperature between *n*-BuLi and *tert*-butyl *N*-(5,6-dichloro-3-pyridyl)carbamate, followed by its 1,2-addition to aldehydes (XVIII) provides secondary alcohols of formula (XIX). Their subsequent oxidation to ketones (XX) using manganese dioxide, followed by a deprotection reaction using organic acids (*e.g.* trifluoroacetic adcid in dichloromethane) provided aminopyridines of formula (XXI). Amides (XXIII) can be obtained by coupling with *N*-Boc protected *L*-amino acids upon exposure to phosphoryl chloride (POCl₃) in pyridine. Removal of *N-Boc* protecting group can be effected with mineral acids (*e.g.* HCl) or organic acids (*e.g.* trifluoroacetic acid) to yield amines of formula (XXIII). Final intramolecular condensation reaction promoted by acidic media (*e.g.* silica in toluene or pivalic acid in ethanol) and heat (80-110 °C) provides desired lactam building block of formula (II).

In alternative, compounds of formula (XXII) can be prepared according to a process described in Scheme 11.

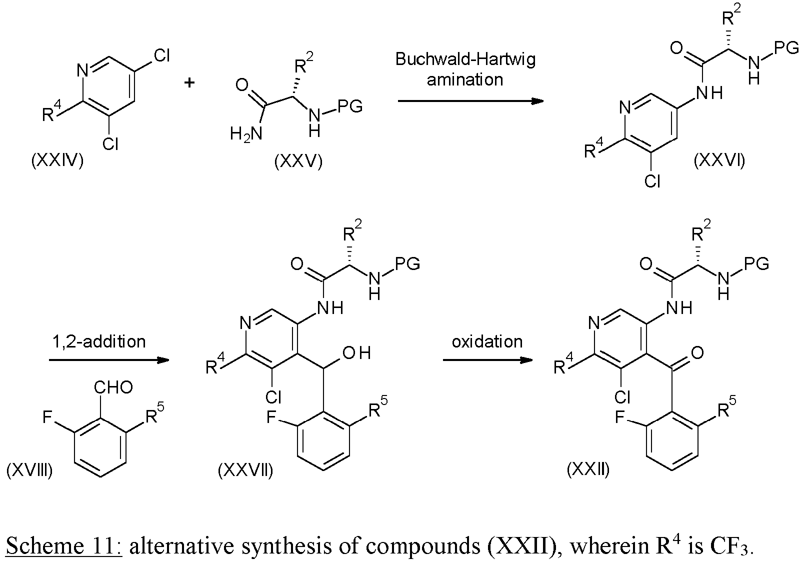

According to Scheme 11, commercially available pyridines (XXIV) can undergo a Buchwald-Hartwig amination reaction with primary amides of formula (XXV), using a palladium catalyst (e.g. Pd₂(dba)₃), a suitable ligand (e.g. Xantphos), and a base such as cesium carbonate. Amides (XXVI) can be deprotonated at low temperature (n-BuLi in tetrahydrofuran at -78°C) to undergo a 1,2-carbonyl addition reaction with commercially available aldehydes (XVIII) to yield alcohols of formula (XXVII). Final oxidation to corresponding ketones (XXII) can be accomplished using TEMPO and sodium hypochlorite.

Notably, in the processes described in schemes 1 to 11, racemization at the chiral center occurs to various extents (20-100%), depending on specific reaction conditions adopted. As a result, chiral purification *(*e.g. by HPLC or SFC) of final derivatives of formula (I), is required to obtain single enantiomers (enantiomeric excess (*ee*) above 97%).

In one aspect, the present invention provides a process of manufacturing the compounds of formula (I) described herein, wherein said process is as described in any one of Schemes 1 to 11 above.

In a further aspect, the present invention provides a compound of formula (I) as described herein, or a pharmaceutically acceptable salt thereof, when manufactured according to the processes disclosed herein.

### Using the Compounds of the Invention

As explained in the background section and illustrated in the experimental section, the compounds of formula (I) and their pharmaceutically acceptable salts possess valuable pharmacological properties that make them useful for the treatment or prevention of diseases or condictions that are associated with the GABAA γ1 receptor.

In one aspect, the present invention provides a compound of formula (I) as described herein, or a pharmaceutically acceptable salt thereof, for use as therapeutically active substance.

In a further aspect, the present invention provides a compound for use in a method for treating or preventing acute neurological disorders, chronic neurological disorders and/or cognitive disorders in a subject, said method comprising administering an effective amount of a compound of formula (I) as described herein, or a pharmaceutically acceptable salt thereof, or a pharmaceutical composition described herein, to the subject.

In a further aspect, the present invention provides a compound of formula (I) as described herein, or a pharmaceutically acceptable salt thereof, or a pharmaceutical composition described herein, for use in a method for treating or preventing acute neurological disorders, chronic neurological disorders and/or cognitive disorders in a subject.

In a further aspect, the present invention provides the use of a compound of formula (I) as described herein, or a pharmaceutically acceptable salt thereof, for the manufacture of a medicament for the treatment of prevention of acute neurological disorders, chronic neurological disorders and/or cognitive disorders.

In one embodiment, said acute neurological disorders, chronic neurological disorders and/or cognitive disorders are selected from autism spectrum disorders (ASD), Angelman syndrome, age-related cognitive decline, Rett syndrome, Prader-Willi syndrome, amyotrophic lateral sclerosis (ALS), fragile-X disorder, negative and/or cognitive symptoms associated with schizophrenia, tardive dyskinesia, anxiety, social anxiety disorder (social phobia), panic disorder, agoraphobia, generalized anxiety disorder, disruptive, impulse-control and conduct disorders, Tourette's syndrome (TS), obsessive-compulsive disorder (OCD), acute stress disorder, post-traumatic stress disorder (PTSD), attention deficit hyperactivity disorder (ADHD), sleep disorders, Parkinson's disease (PD), Huntington's chorea, Alzheimer's disease (AD), mild cognitive impairment (MCI), dementia, behavioral and psychological symptoms (BPS) in neurodegenerative conditions, multi-infarct dementia, agitation, psychosis, substance-induced psychotic disorder, aggression, eating disorders, depression, chronic apathy, anhedonia, chronic fatigue, seasonal affective disorder, postpartum depression, drowsiness, sexual dysfunction, bipolar disorders, epilepsy and pain.

In one embodiment, said acute neurological disorders, chronic neurological disorders and/or cognitive disorders are selected from Alzheimer's disease, mild cognitive impairment (MCI), age-related cognitive decline, negative and/or cognitive symptoms associated with schizophrenia, bipolar disorders, autism spectrum disorder (ASD), Angelman syndrome, Rett syndrome, Prader-Willi syndrome, epilepsy, post-traumatic stress disorder (PTSD), amyotrophic lateral sclerosis (ALS), and fragile-X disorder.

In a preferred embodiment, said acute neurological disorders, chronic neurological disorders and/or cognitive disorders are selected from autism spectrum disorder (ASD), Angelman syndrome, Alzheimer's disease, negative and/or cognitive symptoms associated with schizophrenia and post-traumatic stress disorder (PTSD).

**In** a preferred embodiment, said acute neurological disorders, chronic neurological disorders and/or cognitive disorders are selected from autism spectrum disorder (ASD), Rett syndrome, Angelman syndrome, post-traumatic stress disorder and fragile-X disorder.

**In** a preferred embodiment, said acute neurological disorders, chronic neurological disorders and/or cognitive disorders are selected from autism spectrum disorder (ASD), and Angelman syndrome.

**In** a particularly preferred embodiment, said acute neurological disorders, chronic neurological disorders and/or cognitive disorders are autism spectrum disorder (ASD).

In a further particularly preferred embodiment, said acute neurological disorders, chronic neurological disorders and/or cognitive disorders are Angelman syndrome.

In a further particularly preferred embodiment, said acute neurological disorders, chronic neurological disorders and/or cognitive disorders are autism spectrum disorder (ASD), targeting core symptoms and associated comorbidities, such as anxiety and irritability, social anxiety disorder (social phobia) and generalized anxiety disorder.

### Pharmaceutical Compositions and Administration

In one aspect, the present invention provides pharmaceutical compositions comprising compounds of formula (I) or their pharmaceutically acceptable salts as defined herein and one or more pharmaceutically acceptable excipients. Exemplary pharmaceutical compositions are described in the Example section below.

In a further aspect, the present invention relates to pharmaceutical compositions comprising compounds of formula (I) or their pharmaceutically acceptable salts as defined above and one or more pharmaceutically acceptable excipients for the treatment or prevention of acute neurological disorders, chronic neurological disorders and/or cognitive disorders.

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions or infusion solutions).

The compounds of formula (I) and their pharmaceutically acceptable salts can be processed with pharmaceutically inert, inorganic or organic excipients for the production of tablets, coated tablets, dragées and hard gelatin capsules. Lactose, corn starch or derivatives thereof, talc, stearic acid or its salts etc. can be used, for example, as such excipients for tablets, dragées and hard gelatin capsules.

Suitable excipients for soft gelatin capsules are, for example, vegetable oils, waxes, fats, semi-solid substances and liquid polyols, etc.

Suitable excipients for the production of solutions and syrups are, for example, water, polyols, saccharose, invert sugar, glucose, etc.

Suitable excipients for injection solutions are, for example, water, alcohols, polyols, glycerol, vegetable oils, etc.

Suitable excipients for suppositories are, for example, natural or hardened oils, waxes, fats, semi-solid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Examples

The invention will be more fully understood by reference to the following examples. The claims should not, however, be construed as limited to the scope of the examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

### Example 1

### (7S)-11,12-dichloro-9-(2,6-difluorophenyl)-3,7-dimethyl-2,4,5,8,13-pentazatricyclo[8.4.0.02,6]tetradeca-1(10),3,5,8,11,13-hexaene

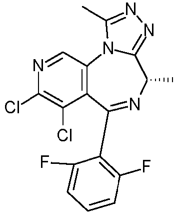

### a) tert-butyl N-tert-butoxycarbonyl-N-(5,6-dichloro-3-pyridyl)carbamate

To a mixture of 5,6-dichloropyridin-3-amine (10 g, 61.3 mmol) in tetrahydrofuran (100 mL) under a nitrogen atmosphere was added *N,N*-diisopropylethylamine (3.97 g, 5.36 mL, 30.7 mmol), di-*tert*-butyl dicarbonate (33.5 g, 35.6 mL, 153 mmol) and 4-dimethylaminopyridine (750 mg, 0.848 ml, 6.13 mmol). The reaction mixture was stirred at room temperature for 18 h. Methyl *tert*-butyl ether (100 mL) was added and the organic layer was washed with aqueous sodium carbonate (1.0 m, 100 mL), water (150 mL) and brine (50 mL). The aqueous layer was extracted with methyl tert-butyl ether (2 × 50 mL). The combined organic layers were dried (MgSO₄) and concentrated *in vacuo* to afford the title compound (23.9 g, 99 %) as a light brown solid. MS: 363.2 ([{³⁵Cl, ³⁵Cl}M+H]⁺), 365.2 ([{³⁵Cl, ³⁷Cl}M+H]⁺), ESI pos.

### b) tert-butyl N-(5,6-dichloro-3-pyridyl)carbamate

To a previously cooled solution (0 °C) of *tert*-butyl *N*-*tert*-butoxycarbonyl-*N*-(5,6-dichloro-3-pyridyl)carbamate (23.93 g, 65.9 mmol) in dichloromethane (226 mL) was slowly added trifluoroacetic acid (12 g, 8.12 ml, 105 mmol). The reaction mixture was stirred at 0 °C for 30 min under nitrogen and allowed to warm up to room temperature overnight. The reaction mixture was quenched with sodium hydrogen carbonate (1.0 m, 150 mL) and stirred for 15 min. The organic layer was washed with sodium hydrogen carbonate (1.0 m, 200 mL). The aqueous layer was extracted with dichloromethane (2 × 200 mL). The combined organic layers were dried (MgSO₄) and concentrated *in vacuo.* The residue was purified by flash chromatography (silica, 0-40 % ethyl acetate in heptane) to afford the title compound (10.5 g, 59 %) as a light yellow solid. MS: 207.0 ([{³⁵Cl, ³⁵Cl}M-C₄H₈-CO₂+H]⁺), 209.1 ([{³⁵Cl, ³⁷Cl}M-C₄H₈-CO₂+H]⁺), ESI pos.

### c) tert-butyl N-[5,6-dichloro-4-[(2,6-difluorophenyl)-hydroxy-methyl]-3-pyridyl]carbamate

A solution of *tert*-butyl (5,6-dichloropyridin-3-yl)carbamate (10.47 g, 39.8 mmol) in anhydrous tetrahydrofuran (108 mL) was cooled down to -70 °C under nitrogen. n-BuLi (2.5 m in hexane, 35 ml, 87.5 mmol) was added dropwise and the mixture was stirred at - 70 °C for 30 min. 2,6-difluorobenzaldehyde (6.79 g, 5.15 ml, 47.8 mmol) was added and the mixture was stirred at -70 °C for 1 h. The reaction mixture was allowed to warm up to - 20 °C, before being quenched by addition of saturated aqueous ammonium chloride (250 mL). The mixture was stirred at 0 °C for 15 min, then further saturated aqueous ammonium chloride (60 mL) was added. The mixture was extracted with methyl *tert*-butyl ether twice, dried (MgSO₄) and concentrated *in vacuo.* The crude was purified by flash chromatography (silica, 0-40 % ethyl acetate in heptane) to afford the title compound (9.21 g, 40 %) as a light yellow solid. MS: 405.2 ([{³⁵Cl, ³⁵Cl}M+H]⁺), 407.2 ([{³⁵Cl, ³⁷Cl}M+H]⁺), ESI pos.

### d) tert-butyl N-[5,6-dichloro-4-(2,6-difluorobenzoyl)-3-pyridyl]carbamate

To a solution of tert-butyl (5,6-dichloro-4-((2,6-difluorophenyl)(hydroxy)methyl)pyridin-3-yl)carbamate (9.21 g, 22.7 mmol) in dichloromethane (500 mL) under nitrogen was added manganese dioxide (22 g, 227 mmol). The reaction mixture was stirred at 50 °C for 3 h, filtered over dicalite, washed with dichloromethane and concentrated *in vacuo.* The residue was purified by flash chromatography (silica, 0-40 % ethyl acetate in heptane) to afford the title compound (9.06 g, 65 %) as a light yellow solid. MS: 347.0 ([{³⁵Cl, ³⁵Cl}M-C₄H₈-CO₂+H]⁺), ESI pos.

### e) (5-amino-2,3-dichloro-4-pyridyl)-(2,6-difluorophenyl)methanone

To a solution of *tert*-butyl *N*-[5,6-dichloro-4-(2,6-difluorobenzoyl)-3-pyridyl]carbamate (9.06 g, 22.5 mmol) in dichloromethane (50 mL) under nitrogen was added trifluoroacetic acid (25.6 g, 17.3 mL, 225 mmol). The reaction mixture was stirred at 25 °C for 3 h, then cooled down to 0 °C (ice bath) and slowly quenched by addition of aqueous sodium carbonate (1.0 m). The organic layer was washed with aqueous sodium carbonate (1.0 m), dried (MgSO₄) and concentrated *in vacuo.* The residue was purified by flash chromatography (silica, 0-50 % ethyl acetate in heptane) to afford the title compound (4.83 g, 55 %) as a yellow solid. MS: 303.1 ([{³⁵Cl, ³⁵Cl}M+H]⁺), 305.1 ([{³⁵Cl, ³⁷Cl}M+H]⁺), ESI pos.

### f) tert-butyl N-[(1S)-2-[[5,6-dichloro-4-(2,6-difluorobenzoyl)-3-pyridyl]amino]-1-methyl-2-oxo-ethyl]carbamate

A solution of (5-amino-2,3-dichloro-4-pyridyl)-(2,6-difluorophenyl)methanone (49 g, 14.8 mmol) in pyridine (43.9 g, 44.9 ml, 556 mmol) was cooled to 0 °C, followed by addition of Boc-Ala-OH (4.76 g, 25.2 mmol) and phosphorous oxychloride (3.41 g, 2.07 mL, 22.2 mmol). The reaction mixture was stirred at 0 °C for 4 h, before being quenched by addition of aqueous sodium hydrogen carbonate (1.0 m, 100 mL). The resulting mixture was extracted with methyl tert-butyl ether (2 × 100 mL) and the organic layers were washed with water (100 mL) and brine (100 mL), dried (MgSO₄) and concentrated *in vacuo.* The residue was purified by flash chromatography (silica, 0-20 % ethyl acetate in heptane) to afford the title compound (4.59 g, 55 %) as an off-white foam. MS: 472.4 ([{³⁵Cl, ³⁵Cl}M-H]⁺), 474.4 ([{³⁵Cl, ³⁷Cl}M-H]⁺), ESI neg.

### g) (2S)-2-amino-N-[5,6-dichloro-4-(2,6-difluorobenzoyl)-3-pyridyl]propanamide

A mixture of *tert-*butyl *N*-[(1*S*)-2-[[5,6-dichloro-4-(2,6-difluorobenzoyl)-3-pyridyl]amino]-1-methyl-2-oxo-ethyl]carbamate (4.51 g, 9.51 mmol) and hydrochloric acid (4.0 m in 1,4-dioxane, 45 mL, 180 mmol) was stirred at room temperature for 2 h. After cooling to 0 °C methyl *tert*-butyl ether (50 mL) was added and the mixture was basified by the addition of aqueous sodium hydrogen carbonate (1.0 m, 250 mL). The aqueous layer was extracted with methyl *tert*-butyl ether (2 × 50 mL), dried (MgSO₄) and concentrated *in vacuo* to afford the title compound (3.15 g, 73 %) as a light brown oil. MS: 374.1 ([{³⁵Cl, ³⁵Cl}M+H]⁺), 376.1 ([{³⁵Cl, ³⁷Cl}M+H]⁺), ESI pos.

### h) (3S)-6,7-dichloro-5-(2,6-difluorophenyl)-3-methyl-1,3-dihydropyrido[3,4-e][1,4]diazepin-2-one

To a mixture of (2S)-2-amino-*N*-[5,6-dichloro-4-(2,6-difluorobenzoyl)-3-pyridyl]propanamide (3.31 g, 8.85 mmol) in toluene (100 mL) was added silica gel (40-63 µm, 15 g, 8.85 mmol). The reaction mixture was stirred at 100 °C for 6 h, then cooled down to room temperature and diluted with ethyl acetate. The mixture was filtered and the silica gel was washed with ethyl acetate (300 mL). The solution was concentrated *in vacuo* and the residue was purified by flash chromatography (silica, 0-50 % ethyl acetate in heptane) to afford the title compound (2.36 g, 75 %) as a yellow solid. MS: 356.1 ([{³⁵Cl, ³⁵Cl}M+H]⁺), 358.1 ([{³⁵Cl, ³⁷Cl}M+H]⁺), ESI pos.

### i) (7S)-11,12-dichloro-9-(2,6-difluorophenyl)-3,7-dimethyl-2,4,5,8,13-pentazatricyclo[8.4.0.02,6]tetradeca-1(10),3,5,8,11,13-hexaene

To a solution of (3*S*)-6,7-dichloro-5-(2,6-difluorophenyl)-3-methyl-1,3-dihydropyrido[3,4-e][1,4]diazepin-2-one (1.91 g, 5.36 mmol) in tetrahydrofuran (764 mL) was added at 0 °C acethydrazide (795 mg, 10.7 mmol), bis(2-oxo-3-oxazolidinyl)phosphinic chloride (2.73 g, 10.7 mmol) and sodium hydride (60 %, 429 mg, 10.7 mmol). After stirring in a thawing ice bath for 18 h, the mixture was stirred at 60 °C for 3 h. After cooling down to room temperature the reaction mixture was diluted with methyl *tert*-butyl ether (50 mL), then treated with aqueous citric acid (5 wt.%, 15 mL). After 15 min, the mixture was basified by addition of aqueous sodium hydrogen carbonate (1.0 m, 50 mL). The aqueous layer was extracted with methyl *tert*-butyl ether (2 × 50 mL). The combined organic layers were dried (MgSO₄) and concentrated *in vacuo.* The residue was purified by flash chromatography (silica, 50-100 % ethyl acetate in heptane) to afford a racemic mixture (1.49 g, 70 %). About 130 mg of this mixture were purified by preparative HPLC (Reprosil Chiral NR, ethanol containing 0.1 % aqueous ammonium acetate / heptane) to afford the enantiopure (-)-title compound (78 mg, 60 %) as an off-white foam. MS: 394.2 ([{³⁵Cl, ³⁵Cl}M+H]⁺), 396.2 ([{³⁵Cl, ³⁷Cl}M+H]⁺), ESI pos.

### Example 2

### (7S)-11-chloro-12-cyclopropyl-9-(2,6-difluorophenyl)-3,7-dimethyl-2,4,5,8,13-pentazatricyclo[8.4.0.02,6]tetradeca-1(10),3,5,8,11,13-hexaene

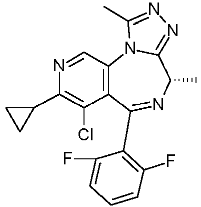

To a solution of (7*S*)-11,12-dichloro-9-(2,6-difluorophenyl)-3,7-dimethyl-2,4,5,8,13-pentazatricyclo[8.4.0.02,6]tetradeca-1(10),3,5,8,11,13-hexaene (93.8 mg, 0.238 mmol) in toluene (1 mL) was added cyclopropylboronic acid (22.5 mg, 0.262 mmol) and potassium phosphate (202 mg, 79 µL, 0.952 mmol). The vial was evacuated and backfilled with argon for three times. After the addition of tricyclohexylphosphine (6.67 mg, 24 µmol) and palladium (II) acetate (2.67 mg, 12 µmol) the vial was capped and filled with argon. The reaction mixture was stirred at 80 °C for 18 h. The reaction was allowed to cool to room temperature, before being filtered through a pad of celite. The filter cake was rinsed with ethyl acetate and the filtrate was concentrated *in vacuo.* The residue was purified by flash chromatography (silica, 0-100 % ethyl acetate in heptane), followed by preparative HPLC (Reprosil Chiral NR, ethanol containing 0.1 % aqueous ammonium acetate / heptane) to afford the enantiopure (-)-title compound (17.5 mg, 18 %) as a colorless oil. MS: 400.1 ([{³⁵Cl}M+H]⁺), 402.1 ([{³⁷Cl}M+H]⁺), ESI pos.

### Example 3

### (7S)-11-chloro-9-(2,6-difluorophenyl)-3,7,12-trimethyl-2,4,5,8,13-pentazatricyclo[8.4.0.02,6]tetradeca-1(10),3,5,8,11,13-hexaene

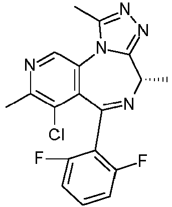

To a solution of (7*S*)-11,12-dichloro-9-(2,6-difluorophenyl)-3,7-dimethyl-2,4,5,8,13-pentazatricyclo[8.4.0.02,6]tetradeca-1(10),3,5,8,11,13-hexaene (107 mg, 0.272 mmol) in 1,4-dioxane (1 mL) was added potassium carbonate (56.4 mg, 0.408 mmol). The vial was evacuated and backfilled with argon for three times. After the addition of tetrakis(triphenylphosphine)palladium(0) (15.7 mg, 13.6 µmol) and trimethylboroxine (37.6 mg, 41.9 µL, 0.299 mmol) the vial was evacuated and backfilled with argon. The reaction mixture was stirred at 80 °C for 18 h. The reaction was allowed to cool to room temperature, before being filtered through a pad of celite. The filter cake was rinsed with ethyl acetate and the filtrate was concentrated *in vacuo.* The residue was purified by flash chromatography (silica, 50-100 % ethyl acetate in heptane, then 0-10 % methanol in ethyl acetate), followed by preparative HPLC (Reprosil Chiral NR, ethanol containing 0.1 % aqueous ammonium acetate / heptane) to afford the enantiopure (-)-title compound (54.6 mg, 67 %) as an off-white foam. MS: 374.2 ([{³⁵Cl}M+H]⁺), 376.2 ([{³⁷Cl}M+H]⁺), ESI pos.

### Example 4

### (7S)-11-chloro-9-(2,6-difluorophenyl)-3,7-dimethyl-12-(trifluoromethyl)-2,4,5,8,13-pentazatricyclo[8.4.0.02,6]tetradeca-1(10),3,5,8,11,13-hexaene

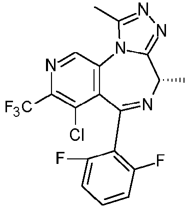

### a) tert-butyl N-[(1S)-2-[[5-chloro-6-(trifluoromethyl)-3-pyridyl]amino]-1-methyl-2-oxoethyl]carbamate

To a solution of 3,5-dichloro-2-(trifluoromethyl)pyridine (5 g, 23.1 mmol) in 1,4-dioxane (74.9 mL) was added cesium carbonate (9.05 g, 27.8 mmol) and *tert*-butyl *N*-[(2*S*)-1-amino-1-oxopropan-2-yl]carbamate (5.23 g, 27.8 mmol). Argon was bubbled through the mixture vigorously. Xantphos (1.34 g, 2.31 mmol) and tris(dibenzylideneacetone)dipalladium (1.06 g, 1.16 mmol) were added and the reaction mixture was stirred at 100 °C for 17 h. The reaction mixture was diluted with dichloromethane and water. The aqueous layer was extracted with dichloromethane. The combined organic layers were dried (Na₂SO₄) and concentrated *in vacuo.* The residue was purified by flash chromatography (silica, 0-55 % ethyl acetate in heptane) to afford the title compound (6.34 g, 73 %) as a white solid. MS: 368.0 ([{³⁵Cl}M+H]⁺), 370.0 ([{³⁷Cl}M+H]⁺), ESI pos.

### b) tert-butyl N-[(1S)-2-[[5-chloro-4-[(2,6-difluorophenyl)-hydroxy-methyl]-6-(trifluoromethyl)-3-pyridyl]amino]-1-methyl-2-oxo-ethyl]carbamate

In analogy to experiment of example 1 c, *tert*-butyl *N*-[(1*S*)-2-[[5-chloro-6-(trifluoromethyl)-3-pyridyl]amino]-1-methyl-2-oxo-ethyl]carbamate was converted into the title compound (8.78 g, 100 %) which was obtained as an orange solid. MS: 510.2 ([{³⁵Cl}M+H]⁺), 512.2 ([{³⁷Cl}M+H]⁺), ESI pos.

### c) tert-butyl N-[(1S)-2-[[5-chloro-4-(2,6-difluorobenzoyl)-6-(trifluoromethyl)-3-pyridyl]amino]-1-methyl-2-oxo-ethyl]carbamate

To a solution of *tert*-butyl *N*-[(1*S*)-2-[[5-chloro-4-[(2,6-difluorophenyl)-hydroxy-methyl]-6-(trifluoromethyl)-3-pyridyl]amino]-1-methyl-2-oxo-ethyl]carbamate ( 8.76 g, 15.3 mmol) in dichloromethane (102 mL) and water (102 mL) was added at 0 °C potassium bromide (2.73 g, 22.9 mmol), sodium hydrogen carbonate (514 mg, 6.12 mmol) and TEMPO (239 mg, 1.53 mmol). Finally, aqueous sodium hypochlorite (10-15 wt.%, 16 ml, 26 mmol) was added dropwise and the reaction mixture was stirred at 0 °C for 2 h. The aqueous layer was extracted with dichloromethane. The combined organic layers were washed with saturated aqueous sodium carbonate and brine, dried (Na₂SO₄) and concentrated *in vacuo.* The residue was purified by flash chromatography (silica, 0-30 % ethyl acetate in heptane) to afford the title compound (4.99 g, 63 %) as a white solid. MS m/e: 508.1 ([{³⁵Cl}M+H]⁺), 510.1 ([{³⁷Cl}M+H]⁺), ESI pos.

### d) (2S)-2-amino-N-[5-chloro-4-(2,6-difluorobenzoyl)-6-(trifluoromethyl)-3-pyridyl]propanamide

In analogy to experiment of example 1 g, *tert-*butyl *N*-[(1*S*)-2-[[5-chloro-4-(2,6-difluorobenzoyl)-6-(trifluoromethyl)-3-pyridyl]amino]-1-methyl-2-oxo-ethyl]carbamate was converted into the title compound (3.36 g, 100 %) which was obtained as an brown oil. MS: 406.0 ([{³⁵Cl}M-H]⁺), 408.1 ([{³⁷Cl}M-H]⁺), ESI neg.

### e) (3S)-6-chloro-5-(2,6-difluorophenyl)-3-methyl-7-(trifluoromethyl)-1,3-dihydropyrido[3,4-e][1,4]diazepin-2-one

In analogy to experiment of example 1 h, (2*S*)-2-amino-*N*-[5-chloro-4-(2,6-difluorobenzoyl)-6-(trifluoromethyl)-3-pyridyl]propanamide was converted into the title compound (2.84 g, 87 %) which was obtained as a yellow solid. MS: 390.0 ([{³⁵Cl}M+H]⁺), 392.0 ([{³⁷Cl}M+H]⁺), ESI pos.

### f) (7S)-11-chloro-9-(2,6-difluorophenyl)-3,7-dimethyl-12-(trifluoromethyl)-2,4,5,8,13-pentazatricyclo[8.4.0.02,6]tetradeca-1(10),3,5,8,11,13-hexaene

In analogy to experiment of example 1 h, (3*S*)-6-chloro-5-(2,6-difluorophenyl)-3-methyl-7-(trifluoromethyl)-1,3-dihydropyrido[3,4-e][1,4]diazepin-2-one was converted into the enantiopure (-)-title compound (118 mg, 49 %) which was obtained as a light yellow solid. MS: 428.2 ([{³⁵Cl}M+H]⁺), 430.1 ([{³⁷Cl}M+H]⁺), ESI pos.

### Example 5

### (7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene

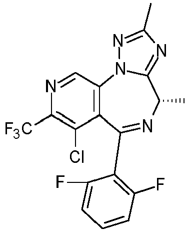

### a) (3S)-6-chloro-5-(2,6-difluorophenyl)-3-methyl-7-(trifluoromethyl)-1,3-dihydropyrido[3,4-e][1,4]diazepine-2-thione

To a mixture of (3*S*)-6-chloro-5-(2,6-difluorophenyl)-3-methyl-7-(trifluoromethyl)-1,3-dihydropyrido[3,4-e][1,4]diazepin-2-one (598 mg, 1.53 mmol) in toluene (10 mL) and 1,4-dioxane (10 mL) was added Lawesson's reagent (372 mg, 0.920 mmol). The yellow suspension was stirred for 29 h at 90 °C. Following addition of a further amount of Lawesson's reagent (372 mg, 0.920 mmol), the mixture was stirred for 68 h. The reaction mixture was allowed to cool to room temperature, before being filtered over 20 g of silica gel. The filter cake was rinsed with toluene (2 × 20 mL) and ethyl acetate (3 × 20 mL). The filtrate was concentrated *in vacuo.* The residue was purified by flash chromatography (silica, 0-25 % ethyl acetate in heptane) to afford the title compound (416 mg, 65 %) as a yellow solid. MS: 404.2 ([{³⁵Cl}M-H]⁺), 406.1 ([{³⁷Cl}M-H]⁺), ESI neg.

### b) (3S)-6-chloro-5-(2,6-difluorophenyl)-3-methyl-7-(trifluoromethyl)-3H-pyrido[3,4-e][1,4]diazepin-2-amine

To a solution of (3*S*)-6-chloro-5-(2,6-difluorophenyl)-3-methyl-7-(trifluoromethyl)-1,3-dihydropyrido[3,4-e][1,4]diazepine-2-thione (124 mg, 0.306 mmol) in tetrahydrofuran (1.84 mL) and methanol (0.707 mL) was added ammonia in methanol (7.0 m, 3.27 ml, 22.9 mmol). The reaction mixture was stirred at 50 °C for 15 h. The reaction mixture was concentrated *in vacuo* and used as such in the following step without further purification. MS: 387.1 ([{³⁵Cl}M-H]⁺), 389.0 ([{³⁷Cl}M-H]⁺), ESI neg.

### c) (7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene

A mixture of (3S)-6-chloro-5-(2,6-difluorophenyl)-3-methyl-7-(trifluoromethyl)-3H-pyrido[3,4-e][1,4]diazepin-2-amine (154 mg, 0.396 mmol) and triethyl orthoacetate (352 mg, 0.398 mL, 2.06 mmol) was stirred at 150 °C for 10 min. The reaction mixture was concentrated at high vacuum to obtain a brown oil. The residue was dissolved in methanol (1 mL), then ammonia in methanol (7.0 m, 57 µL, 0.396 mmol) was added and the reaction was stirred for 25 min. The reaction mixture was concentrated *in vacuo* and the residue was dissolved in methanol (1 mL). Sodium hypochlorite solution (448 mg, 0.372 mL, 0.904 mmol) was added dropwise and the reaction mixture was stirred at room temperature for 30 min, then diluted with water and extracted with dichloromethane. The organic layers were combined, washed with brine, dried (Na₂SO₄) and concentrated *in vacuo.* The residue was purified by flash chromatography (silica, 0-30 % ethyl acetate in heptane), followed by SFC (Chiralcel OD-H, 5 % isopropanol) to afford the enantiopure (-)-enantiopure title compound (8 mg, 6 %) as a light yellow solid. MS m/e: 426.1 ([{³⁵Cl}M+H]⁺), 428.1 ([{³⁷Cl}M+H]⁺), ESI pos.

### Example 6

### azetidin-1-yl-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]methanone

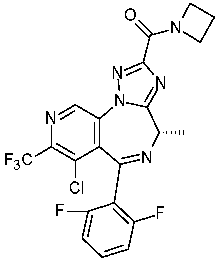

### a) (3S)-1-amino-6-chloro-5-(2,6-difluorophenyl)-3-methyl-7-(trifluoromethyl)-3H-pyrido[3,4-e][1,4]diazepin-2-one

To a solution of (3*S*)-6-chloro-5-(2,6-difluorophenyl)-3-methyl-7-(trifluoromethyl)-1,3-dihydropyrido[3,4-e][1,4]diazepin-2-one (800 mg, 2.05 mmol) in *N,N*-dimethylformamide (20.5 ml) was added (aminooxy)diphenylphosphine oxide (586 mg, 2.46 mmol) and cesium carbonate (1.0 g, 3.08 mmol). The suspension was stirred at 0 °C for 2 h, then concentrated *in vacuo.* The residue was diluted with ethyl acetate (25 mL) and water (25 mL). The aqueous phase was extracted with ethyl acetate (3 × 20 mL). The combined organic layers were washed with brine (20 mL), dried (Na₂SO₄) and concentrated *in vacuo.* The residue was purified by flash chromatography (silica, 0-35 % ethyl acetate in heptane) to afford the title compound (445 mg, 54 %) as a yellow solid. MS: 405.0 ([{³⁵Cl}M+H]⁺), 407.0 ([{³⁷Cl}M+H]⁺), ESI pos.

### b) ethyl (7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate

To a solution of (3*S*)-1-amino-6-chloro-5-(2,6-difluorophenyl)-3-methyl-7-(trifluoromethyl)-3H-pyrido[3,4-e][1,4]diazepin-2-one (386 mg, 0.954 mmol) in toluene (2 mL) was added ethyl 2-ethoxy-2-iminoacetate (415 mg, 2.86 mmol) in toluene (3.2 mL). The reaction mixture was stirred at 80 °C for 2 h, then at 120 °C for 2 h. At this point, p-TsOH monohydrate (181 mg, 0.954 mmol) was added and the reaction mixture was stirred at 120 °C for 23 h. Following addition of a further amount of ethyl 2-ethoxy-2-iminoacetate (138 mg, 0.954 mmol) in toluene (0.8 mL), the reaction was stirred for 4 h. Finally, a further amount of p-TsOH monohydrate (181 mg, 0.954 mmol) and ethyl 2-ethoxy-2-iminoacetate (138.44 mg, 0.954 mmol) in toluene (0.5 mL) were added and the reaction was stirred at 120 °C overnight. Ethyl acetate (20 mL) and saturated aqueous NaHCO₃ (20 mL, 1:1 diluted with water) were added. The aqueous phase was extracted with ethyl acetate (3 × 20 mL). The combined organic phases were washed with brine (3 × 40 mL), dried (Na₂SO₄) and concentrated *in vacuo.* The residue (706 mg, brown oil) was purified by preparative HPLC (Gemini NX, water containing 0.1% formic acid / acetonitrile) to afford the title compound (149 mg, 32 %) as a light brown foam. MS: 486.2 ([{³⁵Cl}M+H]⁺), 488.2 ([{³⁷Cl}M+H]⁺), ESI pos.

### c) (7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid

To a solution of ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate (35 mg, 0.072 mmol) in methanol (0.5 mL) was added sodium hydroxide (11.5 mg, 0.288 mmol). The reaction mixture was stirred at room temperature for 1.5 h, then acidified with aqueous hydrochloric acid (1.0 m, 2 mL). The aqueous layer was extracted with dichloromethane (3 × 5 mL). The combined organic layers were dried (Na₂SO₄) and concentrated *in vacuo* to afford the title compound (28 mg, 83 %) as a yellow solid. The compound was used as such in the following step without further purification. MS: 458.1 ([{³⁵Cl}M+H]⁺), 460.0 ([{³⁷Cl}M+H]⁺), ESI pos.

### d) azetidin-1-yl-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]methanone

To a solution of (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid (28 mg, 0.061 mmol) in *N,N*-dimethylformamide (0.5 mL) was added azetidine hydrochloride (17.17 mg, 0.184 mmol), HATU (27.91 mg, 0.073 mmol) and DIPEA (39.53 mg, 53.27 uL, 0.306 mmol). The reaction mixture was stirred at 40 °C for 16 h, then at 70 °C for 4 h. The reaction mixture was concentrated *in vacuo.* The residue was diluted in ethyl acetate (5 mL) and washed with water (2 × 5 mL). The aqueous phase was extracted with ethyl acetate (2 × 10 mL). The combined organic layers were washed with brine, dried (Na₂SO₄) and concentrated *in vacuo.* The residue was purified by flash chromatography (silica, 40-100 % ethyl acetate in heptane), followed by SFC (Chiralcel OD-H, 20 % methanol) to afford the enantiopure (-)-title compound (3 mg, 3 %) as a white solid. MS: 497.2 ([{³⁵Cl}M+H]⁺), 499.2 ([{³⁷Cl}M+H]⁺), ESI pos.

### Example 7

### [(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-fluoroazetidin-1-yl)methanone

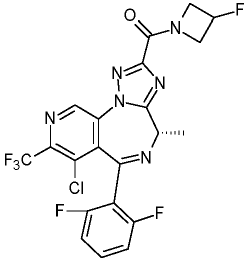

A mixture of 3-fluoroazetidine hydrochloride (230 mg, 2.06 mmol) and sodium carbonate (218 mg, 2.06 mmol) in ethanol (5 mL) was stirred at 15 °C for 10 min. Then ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate (200 mg, 0.41 mmol) was added. The reaction mixture was stirred at 50 °C for 12 h, before being cooled to room temperature. The reaction mixture was diluted with water (10 mL) and extracted with ethyl acetate (3 × 10 mL). The combined organic layers were washed with brine (10 mL), dried (Na₂SO₄) and concentrated *in vacuo.* The residue was purified by preparative HPLC (Waters Xbridge, water containing 0.05 % aqueous ammonia / acetonitrile), followed by SFC (Daicel Chiralpak AS, methanol containing 0.1 % aqueous ammonia) to afford the enantiopure (-)-title compound (55 mg, 17 %) as a white solid. MS: 515.1 ([{³⁵Cl}M+H]⁺), 517.1 ([{³⁷Cl}M+H]⁺), ESI pos.

### Example 8

### [(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxyazetidin-1-yl)methanone

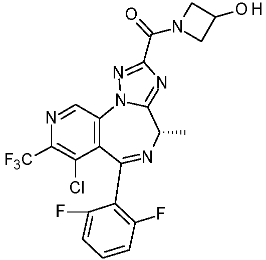

In analogy to experiment of example 7, ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate, using 3-hydroxyazetidine hydrochloride instead of 3-fluoroazetidine hydrochloride, was converted into the enantiopure (-)-title compound (24 mg, 2 %) as a white solid. MS: 513.0 ([{³⁵Cl}M+H]⁺), 515.0 ([{³⁷Cl}M+H]⁺), ESI pos.

### Example 9

### [(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-methoxyazetidin-1-yl)methanone

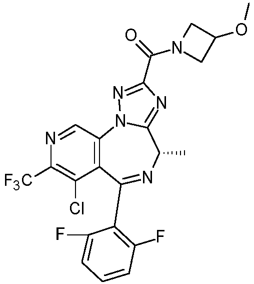

In analogy to experiment of example 7, ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate, using 3-methoxyazetidine hydrochloride instead of 3-fluoroazetidine hydrochloride and trimethylamine instead of sodium carbonate, was converted into the enantiopure (-)-title compound (25 mg, 6 %) as a white solid. MS: 527.0 ([{³⁵Cl}M+H]⁺), 529.0 ([{³⁷Cl}M+H]⁺), ESI pos.

### Example 10

### [(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanone

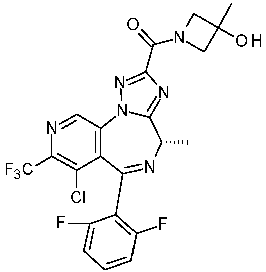

In analogy to experiment of example 7, ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate, using 3-methylazetidin-3-ol hydrochloride instead of 3-fluoroazetidine hydrochloride and trimethylamine instead of sodium carbonate, was converted into the enantiopure (-)-title compound (54 mg, 16 %) as a white solid. MS: 527.2 ([{³⁵Cl}M+H]⁺), 529.2 ([{³⁷Cl}M+H]⁺), ESI pos.

### Example 11

### [(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(1,1-dioxo-1,4-thiazinan-4-yl)methanone

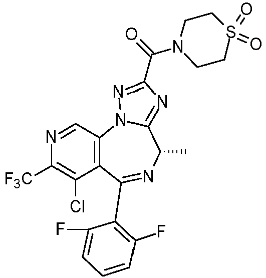

To a mixture of (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid (110 mg, 0.290 mmol) in *N*,*N*-dimethylformamide (2.0 mL) was added benzotriazol-1-yloxytripyrrolidinophosphonium hexafluorophosphate (PyBOP, 150 mg, 0.290 mmol), thiomorpholine 1,1-dioxide hydrochloride (124 mg, 0.720 mmol) and DIPEA (0.25 mL, 1.44 mmol). The reaction mixture was stirred at room temperature for 16 h, then purified by preparative HPLC (Waters Xbridge, water containing 10 mM ammonium hydrogen carbonate / acetonitrile), followed by SFC (Daicel Chiralpak AS, methanol containing 0.1 % aqueous ammonia) to afford the enantiopure (-)-title compound (19.0 mg, 13 %) as an off-white solid. MS: 575.1 ([{³⁵Cl}M+H]⁺), 577.1 ([{³⁷Cl}M+H]⁺), ESI pos.

### Example 12

### N-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]oxetane-3-carboxamide

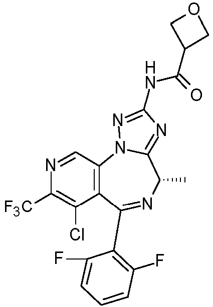

### a) tert-butyl N-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]carbamate

To a mixture of (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid (950 mg, 2.08 mmol) and triethylamine (630 mg, 6.23 mmol) in 1,4-dioxane (10 mL) was slowly added diphenylphosphoryl azide (1.14 g, 4.15 mmol). The mixture was stirred at room temperature for 1 h, then at 50 °C for another 2 h. The mixture was allowed to cool to room temperature, before addition of *tert*-butanol (10 mL). The reaction mixture was stirred at 100 °C for 16 h, before being poured into water (50 mL) and extracted with ethyl acetate (3 × 50 mL). The combined organic layers were washed with brine (50 mL), dried (Na₂SO₄) and concentrated *in vacuo.* The residue was suspended in ethyl acetate, and the precipitate was filtered off. The filtrate was purified by flash chromatography (silica, 20-60 % ethyl acetate in petrol ether) to afford the title compound (370 mg, 34 %) as light brown solid. MS: 473.1 ([{³⁵Cl}M-C₄H₈+H]⁺), ESI pos.

### b) (7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-amine

To a mixture of *tert*-butyl *N*-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]carbamate (370 mg, 0.70 mmol) in dichloromethane (5 mL) was added trifluoroacetic acid (2 mL) slowly. The mixture was stirred at room temperature for 1 h, before addition of saturated aqueous sodium hydrogen carbonate (until pH>8). The mixture was extracted with dichloromethane (3 × 10 mL). The combined organic layers were washed with brine (10 mL), dried (Na₂SO₄) and concentrated *in vacuo* to afford the title compound (298 mg, 99 %) as a light brown solid, which was used as such in the following step without further purification. MS: 429.0 ([{³⁵Cl}M+H]⁺), ESI pos.

### c) N-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]oxetane-3-carboxamide

To a mixture of (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-amine (150.0 mg, 0.350 mmol) and oxetane-3-carboxylic acid (53.6 mg, 0.520 mmol) in pyridine (2 mL) was added phosphoryl chloride (0.05 mL, 0.520 mmol) at 0 °C. The mixture was stirred at 0 °C for 1 h, then poured into ice water (10 mL) and extracted with ethyl acetate (3 × 10 mL). The combined organic layers were washed with water (3 × 10 mL) and brine (10 mL), dried (Na₂SO₄) and concentrated *in vacuo.* The residue was purified by preparative HPLC (Waters Xbridge, water containing 10 mM ammonium hydrogen carbonate / acetonitrile), followed by preparative HPLC (Phenomenex Gemini-NX C18, water containing 0.05 % aqueous ammonia / acetonitrile) and, finally, by SFC (Daicel Chiralcel OJ-H, 25 % isopropanol) to afford the enantiopure (-)-title compound (2.0 mg, 1 %) as a white solid. MS: 513.1 ([{³⁵Cl}M+H]⁺), 515.1 ([{³⁷Cl}M+H]⁺), ESI pos.

### Example 13

### 1-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]pyrrolidin-2-one

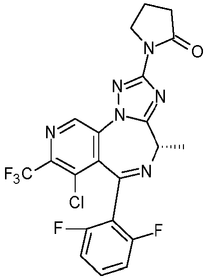

### a) 4-chloro-N-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]butanamide

To a solution of (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-amine (130 mg, 0.30 mmol) and pyridine (757 mg, 5.36 mmol) in acetonitrile (10 mL) was slowly added 4-chlorobutyryl chloride (812 mg, 10.2 mmol) at -20 °C. The mixture was stirred at -20 °C for 18 h, then concentrated *in vacuo.* The residue was diluted with ethyl acetate (10 mL) and washed with water (3 × 5 mL), brine (5 mL), dried (Na₂SO₄) and concentrated *in vacuo* to afford the title compound (200 mg, crude) as a brown oil which was used as such in the following step without further purification. MS: 533.3 ([{³⁵Cl, ³⁵Cl}M+H]⁺), ESI pos.

### b) 1-[(7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]pyrrolidin-2-one

To a solution of 4-chloro-*N*-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]butanamide (200 mg, 0.38 mmol) in *N,N*-dimethylformamide (3 mL) was added triethylamine (0.26 mL, 1.88 mmol). The reaction mixture was stirred at 100 °C for 3 h, then poured into water (10 mL) and extracted with ethyl acetate (3 × 10 mL). The combined organic layers were washed with brine (3 × 10 mL), dried (Na₂SO₄) and concentrated *in vacuo.* The residue was purified by preparative TLC (petrol ether / ethyl acetate 0: 1), followed by preparative HPLC (Waters Xbridge, water containing 10 mM ammonium hydrogen carbonate / acetonitrile), then by SFC (REGIS (s,s) WHELK-O1, 40 % isopropanol) to afford the enantiopure (-)-title compound (8.0 mg, 4 %) as a white solid. MS: 497.1 ([{³⁵Cl}M+H]⁺), 499.1 ([{³⁷Cl}M+H]⁺), ESI pos.

### Example 14

### (7S)-11-chloro-9-(2,6-difluorophenyl)-N-[(2S)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide

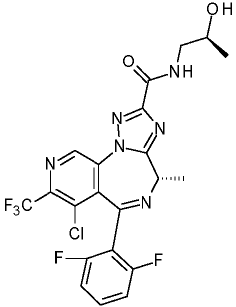

In analogy to experiment of example 7, ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate, using (2*S*)-1-aminopropan-2-ol instead of 3-fluoroazetidine hydrochloride, was converted into the enantiopure (-)-title compound (13.0 mg, 8 %) as a white solid. MS: 515.1 ([{³⁵Cl}M+H]⁺), 517.1 ([{³⁷Cl}M+H]⁺), ESI pos.

### Example 15

### (7S)-11-chloro-9-(2,6-difluorophenyl)-N-(2-hydroxyethyl)-7-methyl-12-(trifluoromethyl)-2,3,5,8,13-pentazatricyclo[8.4.0.02,6]tetradeca-1(10),3,5,8,11,13-hexaene-4-carboxamide

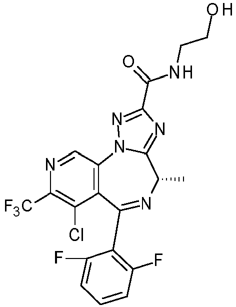

### a) (7S)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid

To a solution of ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate (450 mg, 0.93 mmol) in tetrahydrofuran (2.5 mL) was added triethylamine (2.5 mL, 17.9 mmol) and, slowly, saturated aqueous lithium bromide (2.5 mL). The reaction mixture was stirred at 15 °C for 2 h, then acidified with aqueous hydrochloric acid (1.0 m, 10 mL). The aqueous layer was extracted with ethyl acetate (3 × 10 mL). The combined organic layers were washed with brine (10 mL), dried (Na₂SO₄) and concentrated *in vacuo* to afford the title compound (400 mg, 94 %) as a yellow solid. The compound was used as such in the following step without further purification. MS: 458.0 ([{³⁵Cl}M+H]⁺), 460.0 ([{³⁷Cl}M+H]⁺), ESI pos.

### b) (7S)-11-chloro-9-(2,6-difluorophenyl)-N-(2-hydroxyethyl)-7-methyl-12-(trifluoromethyl)-2,3,5,8,13-pentazatricyclo[8.4.0.02,6]tetradeca-1(10),3,5,8,11,13-hexaene-4-carboxamide

In analogy to experiment of example 6 d, (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid, using 2-aminoethanol instead of azetidine hydrochloride, was converted into the enantiopure (-)-title compound (111 mg, 23 %) as an off-white solid. MS: 501.1 ([{³⁵Cl}M+H]⁺), 503.1 ([{³⁷Cl}M+H]⁺), ESI pos.

### Example 16

### (7S)-11-chloro-9-(2,6-difluorophenyl)-N-[(2R)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide

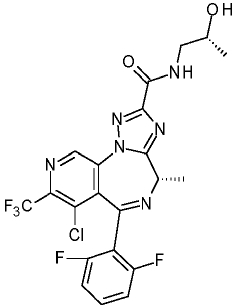

In analogy to experiment of example 7, ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate, using (2R)-1-aminopropan-2-ol instead of 3-fluoroazetidine hydrochloride, was converted into the enantiopure (-)-title compound (16 mg, 15 %) as an off-white solid. MS: 515.4 ([{³⁵Cl}M+H]⁺), 517.4 ([{³⁷Cl}M+H]⁺), ESI pos.

### Example 17

### (7S)-11-chloro-9-(2-chloro-6-fluoro-phenyl)-3,7-dimethyl-12-(trifluoromethyl)-2,4,5,8,13-pentazatricyclo[8.4.0.02,6]tetradeca-1(10),3,5,8,11,13-hexaene

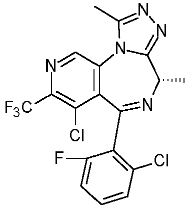

### a) tert-butyl N-[(1S)-2-[[5-chloro-4-[(2-chloro-6-fluoro-phenyl)-hydroxy-methyl]-6-(trifluoromethyl)-3-pyridyl]amino]-1-methyl-2-oxo-ethyl]carbamate

In analogy to experiment of example 1 c, *tert*-butyl *N*-[(1*S*)-2-[[5-chloro-6-(trifluoromethyl)-3-pyridyl]amino]-1-methyl-2-oxo-ethyl]carbamate was converted into the title compound (2.2 g, 38 %) which was obtained as a yellow solid. MS: 510.2 ([{³⁵Cl, ³⁵Cl}M+H]⁺), 512.2 ([{³⁵Cl, ³⁷Cl}M+H]⁺), ESI pos.

### b) tert-butyl N-[(1S)-2-[[5-chloro-4-(2-chloro-6-fluoro-benzoyl)-6-(trifluoromethyl)-3-pyridyl]amino]-1-methyl-2-oxo-ethyl]carbamate

In analogy to experiment of example 1 d, *tert*-butyl *N*-[(1*S*)-2-[[5-chloro-4-[(2-chloro-6-fluoro-phenyl)-hydroxy-methyl]-6-(trifluoromethyl)-3-pyridyl]amino]-1-methyl-2-oxoethyl]carbamate was converted into the title compound (1.7 g, 85 %) which was obtained as a yellow solid. MS: 524.0 ([{³⁵Cl, ³⁵Cl}M+H]⁺), 526.0 ([{³⁵Cl, ³⁷Cl}M+H]⁺), ESI pos.

### c) (2S)-2-amino-N-[5-chloro-4-(2-chloro-6-fluoro-benzoyl)-6-(trifluoromethyl)-3-pyridyl]propanamide

In analogy to experiment of example 1 g, *tert*-butyl *N*-[(1*S*)-2-[[5-chloro-4-(2-chloro-6-fluoro-benzoyl)-6-(trifluoromethyl)-3-pyridyl]amino]-1-methyl-2-oxo-ethyl]carbamate was converted into the title compound (1.3 g, 95 %) which was obtained as a yellow oil. MS: 423.9 ([{³⁵Cl, ³⁵Cl}M+H]⁺), 425.9 ([{³⁵Cl, ³⁷Cl}M+H]⁺), ESI pos.

### d) (3S)-6-chloro-5-(2-chloro-6-fluoro-phenyl)-3-methyl-7-(trifluoromethyl)-1,3-dihydropyrido[3,4-e][1,4]diazepin-2-one

In analogy to experiment of example 1 h, (2*S*)-2-amino-*N*-[5-chloro-4-(2-chloro-6-fluorobenzoyl)-6-(trifluoromethyl)-3-pyridyl]propanamide was converted into the title compound (420 mg, 34 %) which was obtained as a yellow oil. MS: 405.9 ([{³⁵Cl, ³⁵Cl}M+H]⁺), 407.9 ([{³⁵Cl, ³⁷Cl}M+H]⁺), ESI pos.

### e) (3S)-6-chloro-5-(2-chloro-6-fluoro-phenyl)-3-methyl-7-(trifluoromethyl)-1,3-dihydropyrido[3,4-e][1,4]diazepine-2-thione

In analogy to experiment of example 5 a, (3*S*)-6-chloro-5-(2-chloro-6-fluoro-phenyl)-3-methyl-7-(trifluoromethyl)-1,3-dihydropyrido[3,4-e][1,4]diazepin-2-one was converted into the title compound (110 mg, 56 %) which was obtained as a yellow foam. MS: 421.9 ([{³⁵Cl, ³⁵Cl}M+H]⁺), 423.9 ([{³⁵Cl, ³⁷Cl}M+H]⁺), ESI pos.

### f) (7S)-11-chloro-9-(2-chloro-6-fluoro-phenyl)-3,7-dimethyl-12-(trifluoromethyl)-2,4,5,8,13-pentazatricyclo[8.4.0.02,6]tetradeca-1(10),3,5,8,11,13-hexaene

To a mixture of (3*S*)-6-chloro-5-(2-chloro-6-fluoro-phenyl)-3-methyl-7-(trifluoromethyl)-1,3-dihydropyrido[3,4-e][1,4]diazepine-2-thione (100 mg, 0.24 mmol) in 1-butanol (0.5 mL) was added acetohydrazide (35.1 mg, 0.47 mmol). The reaction mixture was stirred at 120 °C for 16 h, before being cooled to room temperature and concentrated *in vacuo.* The residue was purified by preparative HPLC (Waters Xbridge, water containing 0.05 % aqueous ammonia / acetonitrile), followed by SFC (Daicel Chiralcel OD, methanol containing 0.1 % aqueous ammonia) to afford the enantiopure (-)-title compound (9.0 mg, 9 %) as a light yellow solid. MS: 444.1 ([{³⁵Cl, ³⁵Cl}M+H]⁺), 446.1 ([{³⁵Cl, ³⁷Cl}M+H]⁺), ESI pos.

### Example 18

### (7S)-11-chloro-9-(2,6-difluorophenyl)-4,7-dimethyl-12-(trifluoromethyl)-2,5,8,13-tetrazatricyclo[8.4.0.02,6]tetradeca-1(10),3,5,8,11,13-hexaene

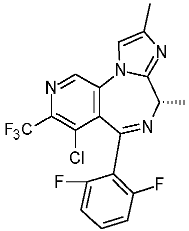

### a) 2-[(E/Z)-[6-chloro-5-(2,6-difluorophenyl)-3-methyl-7-(trifluoromethyl)-1,3-dihydropyrido[3,4-e][1,4]diazepin-2-ylidene]amino]propan-1-ol

To a mixture of sodium carbonate (180 mg, 1.7 mmol) in ethanol (7.2 mL) and water (3.6 mL) was added (3*S*)-6-chloro-5-(2,6-difluorophenyl)-3-methyl-7-(trifluoromethyl)-1,3-dihydropyrido[3,4-e][1,4]diazepine-2-thione (300 mg, 0.740 mmol) and 2-aminopropan-1-ol (111 mg, 1.48 mmol). The reaction mixture was stirred at 80 °C for 12 h, then concentrated *in vacuo.* The residue was purified by flash chromatography (C18, water containing 0.1% formic acid / acetonitrile) to afford the title compound (130 mg, 39 %) as a yellow solid. MS: 447.0 ([{³⁵Cl}M+H]⁺), 449.0 ([{³⁷Cl}M+H]⁺), ESI pos.

### b) (7S)-11-chloro-9-(2,6-difluorophenyl)-4,7-dimethyl-12-(trifluoromethyl)-2,5,8,13-tetrazatricyclo[8.4.0.02,6]tetradeca-1(10),3,5,8,11,13-hexaene

To a mixture of 2-[(*E*/*Z*)-[6-chloro-5-(2,6-difluorophenyl)-3-methyl-7-(trifluoromethyl)-1,3-dihydropyrido[3,4-e][1,4]diazepin-2-ylidene]amino]propan-1-ol (110 mg, 0.250 mmol) and sodium hydrogen carbonate (83 mg, 0.98 mmol) in dichloromethane (6 mL) was added Dess-Martin periodane (157 mg, 0.370 mmol). The reaction mixture was stirred at room temperature for 1 h, then poured into water (10 mL) and extracted with dichloromethane (3 × 10 mL). The combined organic layers were washed with brine (10 mL), dried (Na₂SO₄) and concentrated *in vacuo.* The residue was purified by preparative HPLC (Waters Xbridge, water containing 10 mM ammonium hydrogen carbonate / acetonitrile), followed by SFC (Phenomenex-Cellulose-2, isopropanol) to afford the enantiopure (-)-title compound (1.1 mg, 1 %) as a white solid. MS: 427.1 ([{³⁵Cl}M+H]⁺), 429.1 ([{³⁷Cl}M+H]⁺), ESI pos.

### Example 19

### (7S)-11-chloro-9-(2,6-difluorophenyl)-N-(2-hydroxyethyl)-7-methyl-12-(trifluoromethyl)-2,5,8,13-tetrazatricyclo[8.4.0.02,6]tetradeca-1(10),3,5,8,11,13-hexaene-4-carboxamide

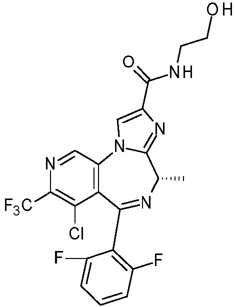

### a) [(3S)-6-chloro-5-(2,6-difluorophenyl)-3-methyl-7-(trifluoromethyl)-3H-pyrido[3,4-e][1,4]diazepin-2-yl] diphenyl phosphate

To a mixture of (3*S*)-6-chloro-5-(2,6-difluorophenyl)-3-methyl-7-(trifluoromethyl)-1,3-dihydropyrido[3,4-e][1,4]diazepin-2-one (500 mg, 1.3 mmol) in tetrahydrofuran (5 mL) was added portion wise sodium hydride (103 mg, 2.6 mmol) at 0 °C. The mixture was stirred for 15 min, then [chloro(phenoxy)phosphoryl]oxybenzene (517 mg, 2 mmol) was slowly added at 0 °C. The reaction mixture was stirred at 0 °C for another 1 h, diluted with water (50 mL) and extracted with ethyl acetate (3 × 30 mL). The combined organic layers were washed with brine (2 × 30 mL), dried (Na₂SO₄) and concentrated *in vacuo.* The residue was purified by flash chromatography (silica, petrol ether / ethyl acetate 3:1) to afford the title compound (300 mg, 22 %) as a yellow solid. MS: 621.9 ([{³⁵Cl}M+H]⁺), 623.9 ([{³⁷Cl}M+H]⁺), ESI pos.

### b) ethyl 2-[[(3S)-6-chloro-5-(2,6-difluorophenyl)-3-methyl-7-(trifluoromethyl)-3H-pyrido[3,4-e][1,4]diazepin-2-yl]amino]-3-hydroxy-propanoate

To a solution of ethyl 2-amino-3-hydroxy-propanoate hydrochloride (409 mg, 2.4 mmol) in tetrahydrofuran (5 mL) was added triethylamine (0.34 mL, 2.4 mmol). The mixture was stirred at 15 °C for 20 min, then [(3*S*)-6-chloro-5-(2,6-difluorophenyl)-3-methyl-7-(trifluoromethyl)-3H-pyrido[3,4-e][1,4]diazepin-2-yl] diphenyl phosphate (500 mg, 0.8 mmol) was added at -20 °C. The reaction mixture was allowed to warm up to 15 °C and stirred for 16 h. The mixture was slowly poured into saturated aqueous ammonium chloride (50 mL) and diluted with water (50 mL). The mixture was extracted with ethyl acetate (3 x 30 mL). The combined organic layers were washed with brine (20 mL), dried (Na₂SO₄) and concentrated *in vacuo.* The residue was purified by preparative TLC (silica, dichloromethane / methanol 20:1) to afford the title compound (300 mg, 65 %) as a yellow solid. MS: 505.0 ([{³⁵Cl}M+H]⁺), 507.0 ([{³⁷Cl}M+H]⁺), ESI pos.

### c) ethyl (7S)-11-chloro-9-(2,6-difluorophenyl)-7-methyl-12-(trifluoromethyl)-2,5,8,13-tetrazatricyclo[8.4.0.02,6]tetradeca-1(10),3,5,8,11,13-hexaene-4-carboxylate

In analogy to experiment of example 18 b, ethyl 2-[[(3*S*)-6-chloro-5-(2,6-difluorophenyl)-3-methyl-7-(trifluoromethyl)-3H-pyrido[3,4-e][1,4]diazepin-2-yl]amino]-3-hydroxy-propanoate was converted into the title compound (100 mg, 33 %) which was obtained as a yellow solid. MS: 485.0 ([{³⁵Cl}M+H]⁺), 487.0 ([{³⁷Cl}M+H]⁺), ESI pos.

### d) (7S)-11-chloro-9-(2,6-difluorophenyl)-7-methyl-12-(trifluoromethyl)-2,5,8,13-tetrazatricyclo[8.4.0.02,6]tetradeca-1(10),3,5,8,11,13-hexaene-4-carboxylic acid

In analogy to experiment of example 15 a, ethyl (7*S*)-11-chloro-9-(2,6-difluorophenyl)-7-methyl-12-(trifluoromethyl)-2,5,8,13-tetrazatricyclo[8.4.0.02,6]tetradeca-1(10),3,5,8,11,13-hexaene-4-carboxylate was converted into the title compound (70 mg, 68 %) which was obtained as a yellow solid. MS: 457.0 ([{³⁵Cl }M+H]⁺), 459.0 ([{³⁷Cl}M+H]⁺), ESI pos.

### e) (7S)-11-chloro-9-(2,6-difluorophenyl)-N-(2-hydroxyethyl)-7-methyl-12-(trifluoromethyl)-2,5,8,13-tetrazatricyclo[8.4.0.02,6]tetradeca-1(10),3,5,8,11,13-hexaene-4-carboxamide

In analogy to experiment of example 6 d, (7*S*)-11-chloro-9-(2,6-difluorophenyl)-7-methyl-12-(trifluoromethyl)-2,5,8,13-tetrazatricyclo[8.4.0.02,6]tetradeca-1(10),3,5,8,11,13-hexaene-4-carboxylic acid, using 2-aminoethanol instead of azetidine hydrochloride, was converted into the enantiopure (-)-title compound (1.0 mg, 2 %) as a white solid. MS: 500.1 ([{³⁵Cl}M+H]⁺), 502.1 ([{³⁷Cl}M+H]⁺), ESI pos.

### Example 20

### (10S)-6-chloro-8-(2,6-difluorophenyl)-10-methyl-5-(trifluoromethyl)-1,4,9,12-tetrazatetracyclo[9.6.0.02,7.013,17]heptadeca-2(7),3,5,8,11,13(17)-hexaene

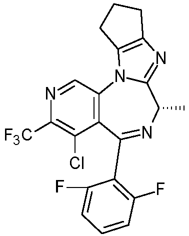

### a) 2-[(E,Z)-[6-chloro-5-(2,6-difluorophenyl)-3-methyl-7-(trifluoromethyl)-1,3-dihydropyrido[3,4-e][1,4]diazepin-2-ylidene]amino]cyclopentanol

To a mixture of sodium carbonate (240.3 mg, 2.27 mmol) in *tert*-butanol (5 mL) was added (3*S*)-6-chloro-5-(2,6-difluorophenyl)-3-methyl-7-(trifluoromethyl)-1,3-dihydropyrido[3,4-e][1,4]diazepine-2-thione (400 mg, 0.990 mmol), followed by 2-aminocyclopentanol (199 mg, 1.97 mmol). The reaction mixture was stirred at 100 °C for 12 h, then poured into water (20 mL) and extracted with ethyl acetate (3 × 20 mL). The combined organic layers were washed by brine (20 mL), dried (Na₂SO₄) and concentrated *in vacuo.* The residue was purified by flash chromatography (silica, 40-60 % ethyl acetate in petrol ether) to afford the title compound (450 mg, 97 %) as a yellow foam. MS: 473.1 ([{³⁵Cl}M+H]⁺), 475.1 ([{³⁷Cl}M+H]⁺), ESI pos.

### b) 2-[(E/Z)-[6-chloro-5-(2,6-difluorophenyl)-3-methyl-7-(trifluoromethyl)-1,3-dihydropyrido[3,4-e][1,4]diazepin-2-ylidene]amino]cyclopentanone

To a mixture of 2-[(*E*/*Z*)-[6-chloro-5-(2,6-difluorophenyl)-3-methyl-7-(trifluoromethyl)-1,3-dihydropyrido[3,4-e][1,4]diazepin-2-ylidene]amino]cyclopentanol (300.0 mg, 0.630 mmol) in dichloromethane (6 mL) was added phenyl-λ3-iodanediyl diacetate (BAIB, 817 mg, 2.54 mmol) and (2,2,6,6-tetramethylpiperidin-1-yl)oxidanyl (TEMPO, 198 mg, 1.27 mmol). The reaction mixture was stirred at 30 °C for 4 h, then poured into water and extracted with dichloromethane. The organic layer was concentrated *in vacuo* and the residue was purified by flash chromatography (C18, water containing formic acid / acetonitrile) to afford the title compound (140 mg, 0.30 mmol, 47 %) as a yellow gum. MS: 471.1 ([{³⁵Cl}M+H]⁺), 473.1 ([{³⁷Cl}M+H]⁺), ESI pos.

### c) (10S)-6-chloro-8-(2,6-difluorophenyl)-10-methyl-5-(trifluoromethyl)-1,4,9,12-tetrazatetracyclo[9.6.0.02,7.013,17]heptadeca-2(7),3,5,8,11,13(17)-hexaene

To a mixture of 2-[(*E*/*Z*)-[6-chloro-5-(2,6-difluorophenyl)-3-methyl-7-(trifluoromethyl)-1,3-dihydropyrido[3,4-e][1,4]diazepin-2-ylidene]amino]cyclopentanone (140 mg, 0.30 mmol) in pyridine (2 mL) was added POCl₃ (228 mg, 1.49 mmol). The reaction mixture was stirred at 25 °C for 1 h, then poured into ice-water (10 mL) and extracted with ethyl acetate. The combined organic layers were dried (Na₂SO₄) and concentrated *in vacuo.* The residue was purified by preparative HPLC (Waters Xbridge, water containing aqueos ammonia / acetonitrile), followed by SFC (REGIS(S,S)WHELK-O1, methanol) to afford the enantiopure (-)-title compound (16 mg, 9 %) as a white solid. MS: 453.1 ([{³⁵Cl}M+H]⁺), 455.1 ([{³⁷Cl}M+H]⁺), ESI pos.

### Example 21

### (7S)-11-chloro-9-(2,6-difluorophenyl)-N-(2-hydroxy-2-methyl-propyl)-7-methyl-12-(trifluoromethyl)-2,3,5,8,13-pentazatricyclo[8.4.0.02,6]tetradeca-1(10),3,5,8,11,13-hexaene-4-carboxamide

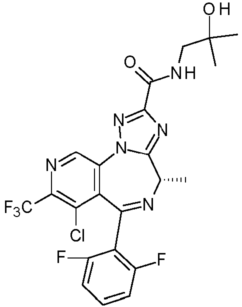

In analogy to experiment of example 7, ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate, using 1-amino-2-methyl-propan-2-ol instead of 3-fluoroazetidine hydrochloride, was converted into the enantiopure (-)-title compound (118 mg, 36 %) as a white solid. MS: 529.2 ([{³⁵Cl}M+H]⁺), 531.2 ([{³⁷Cl}M+H]⁺), ESI pos.

### Example 22

### (7S)-11-chloro-9-(2,6-difluorophenyl)-N-[(1-hydroxycyclopropyl)methyl]-7-methyl-12-(trifluoromethyl)-2,3,5,8,13-pentazatricyclo[8.4.0.02,6]tetradeca-1(10),3,5,8,11,13-hexaene-4-carboxamide

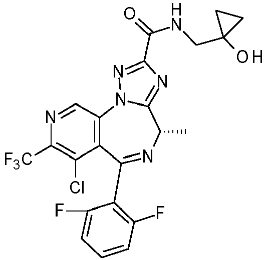

In analogy to experiment of example 7, ethyl (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylate, using 1-(aminomethyl)cyclopropanol instead of 3-fluoroazetidine hydrochloride, was converted into the enantiopure (-)-title compound (86 mg, 27 %) as a yellow solid. MS: 527.1 ([{³⁵Cl}M+H]⁺), 529.1 ([{³⁷Cl}M+H]⁺), ESI pos.

### Example 23

### (7S)-11-chloro-9-(2,6-difluorophenyl)-N-cis-(3-hydroxycyclobutyl)-7-methyl-12-(trifluoromethyl)-2,3,5,8,13-pentazatricyclo[8.4.0.02,6]tetradeca-1(10),3,5,8,11,13-hexaene-4-carboxamide

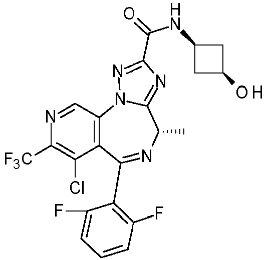

In analogy to experiment of example 6 d, (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid, using *cis*-3-aminocyclobutanol hydrochloride instead of azetidine hydrochloride, was converted into the enantiopure (-)-title compound (7.1 mg, 6 %) which was obtained as a yellow solid. MS: 527.1 ([{³⁵Cl}M+H]⁺), 529.1 ([{³⁷Cl}M+H]⁺), ESI pos.

### Example 24

### (7S)-11-chloro-9-(2,6-difluorophenyl)-N-trans-(3-hydroxycyclobutyl)-7-methyl-12-(trifluoromethyl)-2,3,5,8,13-pentazatricyclo[8.4.0.02,6]tetradeca-1(10),3,5,8,11,13-hexaene-4-carboxamide

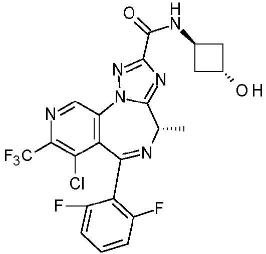

In analogy to experiment of example 6 d, (7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaene-4-carboxylic acid, using trans-3-aminocyclobutanol hydrochloride instead of azetidine hydrochloride, was converted into the enantiopure (-)-title compound (27 mg, 11 %) which was obtained as an off-white solid. MS: 527.1 ([{³⁵Cl}M+H]⁺), 529.1 ([{³⁷Cl}M+H]⁺), ESI pos.

### Assay procedures

### Membrane preparation and binding assay for γ1-containing GABA_{A} subtypes

The affinity of compounds at GABA_{A} γ1 subunit-containing receptors was measured by competition for [³H]RO7239181 (67.3 Ci/mmol; Roche) binding to membranes from HEK293F cells (ThermoFisher R79007) expressing human (transiently transfected) receptors of composition α5β2γ1, α2β2γ1, α1β2γ1. For better protein expression of the α2 subunit-containing receptors, the 28 amino acid long signal peptide (Met1 to Ala28)of the human GABA_{A} α2 subunit was substituted by the 31 amino acid long signal peptide (Met1 to Ser31) of human GABA_{A} α5 subunit.

Harvested pellets from HEK293F cells expressing the different GABA_{A} receptor subtypes were resuspended in Mannitol Buffer pH 7.2-7.4 (Mannitol 0.29 M, Triethylamine 10 mM, Acetic acid 10 mM, EDTA 1mM plus protease inhibitors (20 tablets Complete, Roche Diagnostics Cat. No. 05 056 489 001 per liter)), washed two times and then resuspended at 1:10 to 1:15 dilution in the same buffer. Cell disruption was performed by stirring the suspension in a Parr vessel #4637 at 435 psi for 15 minutes, and then the suspensions were centrifuged at 1000xg for 15 minutes at 4°C (Beckman Avanti J-HC; rotor JS-4.2). The supernatant (S1) was transferred in a 21 Schott flask and the pellet (P1) was resuspended with Mannitol Buffer up to 175ml. The resuspended pellet was transferred into a 250ml Corning centrifugal beaker and centrifuged at 1500xg for 10 minutes at 4°C (Beckman Avanti J-HC; rotor JS-4.2). The supernatant (S1) was then transferred in the 21 Schott flask and the pellet was discarded. The supernatants (S1) were centrifuged in 500ml Beckman polypropylene centrifugal beaker at 15'000xg for 30 minutes at 4°C (Beckman Avanti J-20 XP; rotor JLA-10.500). The pellet (P2) was resuspended with Mannitol Buffer 1:1 and frozen at -80°C. The supernatant (S2) was centrifuged in 100 ml Beckman polypropylene centrifugal tubes at 48000xg for 50 minutes at 4°C (Beckman Avanti J-20 XP; rotor JA-18). The supernatant (S3) was discarded and the pellet (P3) was resuspended with 1:1 Mannitol Buffer. The P2 and P3 protein concentration was determined with the BIORAD Standard assay method with bovine serum albumin as standard and measured on the NANO-Drop 1000. The membrane suspension was aliquots (500µl per tube) and stored at -80°C until required.

Membrane homogenates were resuspended and polytronised (Polytron PT1200E Kinematica AG) in Potassium Phosphate 10 mM, KCl 100 mM binding buffer at pH 7.4 to a final assay concentration determined with a previous experiment.

Radioligand binding assays were carried out in a volume of 200 µL (96-well plates) which contained 100 µL of cell membranes, [³H]RO7239181 at a concentration of 1.5 nM (α5β2γ1) or 20-30 nM (α1β2γ1, α2β2γ1) and the test compound in the range of [0.3-10000] × 10⁻⁹ M. Nonspecific binding was defined by 10 × 10⁻⁶ (α5β2γ1) and 30 × 10⁻⁶ M RO7239181 and typically represented less than 5% (α5β2γ1) and less than 20% (α1β2γ1, α2β2γ1) of the total binding. Assays were incubated to equilibrium for 1 hour at 4 °C and then, membranes were filtered onto unifilter (96-well white microplate with bonded GF/C filters preincubated 20-50 minutes in 0.3% Polyethylenimine) with a Filtermate 196 harvester (Packard BioScience) and washed 4 times with cold Potassium Phosphate 10 mM pH 7.4, KCl 100 mM binding buffer. After anhydrousing, filter-retained radioactivity was detected by liquid scintillation counting. Kᵢ values were calculated using Excel-Fit (Microsoft) and are the means of two determinations.

The compounds of the accompanying examples were tested in the above described assays, and the preferred compounds were found to possess a Kᵢ value for the displacement of [³H]RO7239181 from GABA_{A} γ1 subunit-containing receptors (e.g. α5β2γ1, α2β2γ1, α1β2γ1) of 100 nM or less. Most preferred are compounds with a Ki (nM) < 50. Representative test results, obtained by the above described assay measuring binding affinity to HEK293 cells expressing human (h) receptors, are shown in the Table 1.

### Preparation of [³H]RO7239181, 6-chloro-5-(2,6-difluorophenyl)-7-methyl-1-(tritritiomethyl)-3H-1,4-benzodiazepin-2-one

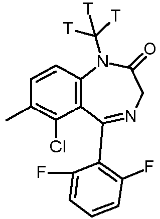

### a) 5-chloro-2-methyl-3,1-benzoxazin-4-one

A solution of 2-amino-6-chlorobenzoic acid (250 g, 1.46 mol) in acetic anhydride (1250 mL) was stirred at 140 °C for 2 h. The reaction mixture was concentrated in vacuo. The resulting crude residue was suspended in ethyl acetate (1000 mL), stirred for 30 min, filtered and dried in vacuo to afford the title compound (238 g, 84 %) as a grey solid. ¹H NMR (DMSO-d6, 400 MHz): δ: 7.80 (app t, *J =* 8.0 Hz, 1H), 7.62 (d, *J =* 8.0 Hz, 1H), 7.49 (d, *J =* 7.6 Hz, 1H), 2.36 (s, 3H).

### b) N-[3-chloro-2-(2,6-difluorobenzoyl)phenyl]acetamide

To a solution of 5-chloro-2-methyl-3,1-benzoxazin-4-one (100 g, 511.2 mmol) and 2-bromo-1,3-difluorobenzene (118.4 g, 613.5 mmol) in tetrahydrofuran (1000 mL) was added dropwise *i*-PrMgCl·LiCl (1.3 M, 500 mL, 650 mmol) at -70 °C under nitrogen. The mixture was allowed to warm up to room temperature within 1 h, quenched with saturated aqueous ammonium chloride (1500 mL) and extracted with ethyl acetate (2 x 1500 mL). The organic phase was washed with brine (2000 mL), dried (Na₂SO₄) and concentrated in vacuo. The residue was suspended in ethyl acetate (150 mL). The resulting suspension was stirred at room temperature for 20 min, filtered and dried in vacuo to afford the title compound (113 g, 71 %) as an off-white solid. ¹H NMR (DMSO-d6, 400 MHz): δ: 9.85 (s, 1H), 7.65-7.45 (m, 1H), 7.40 (t, *J =* 7.2 Hz, 1H), 7.38-7.34 (m, 2H), 7.16 (t, *J =* 8.8 Hz, 2H), 1.85 (s, 3H).

### c) (2-amino-6-chloro-phenyl)-(2,6-difluorophenyl)methanone

To a solution of *N*-[3-chloro-2-(2,6-difluorobenzoyl)phenyl]acetamide (113 g, 364.9 mmol) in ethanol (250 mL) was added aqueous hydrochloric acid (12 M, 200 mL). The reaction mixture was stirred at 100 °C for 1 h, then diluted with ethyl acetate (1100 mL). The organic phase was washed with water (1100 mL), saturated aqueous sodium bicarbonate (1100 mL) and brine (1100 mL), dried over sodium sulfate and concentrated in vacuo. Petroleum ether (120 mL) was added to the crude and the suspension was stirred at room temperature for 20 min. The solid was filtered and dried to afford the title compound (88 g, 90 %) as a yellow solid. ¹H NMR (DMSO-d6, 400 MHz): δ: 7.62-7.56 (m, 1H), 7.21-7.15 (m, 3H), 6.83 (d, *J =* 7.6 Hz, 1H), 6.74 (s, 2H), 6.58 (d, *J =* 7.6 Hz, 1H).

### d) (6-amino-3-bromo-2-chloro-phenyl)-(2,6-difluorophenyl)methanone

To a solution of (2-amino-6-chloro-phenyl)-(2,6-difluorophenyl)methanone (88.0 g, 328.8 mmol) in dichloromethane (225 mL) and *N,N-*dimethylformamide (225 mL) was added 1-bromopyrrolidine-2,5-dione (64.4 g, 362 mmol) at 0 °C. The reaction mixture was stirred at 30 °C for 1 h. The mixture was diluted with dichloromethane (600 mL) and washed with water (500 mL) and brine (4 × 500 mL), dried (Na₂SO₄) and concentrated in vacuo. The residue was purified by chromatography (silica, petroleum ether / ethyl acetate, 1:0 to 2:1). The solid was suspended in petroleum ether (200 mL) and stirred at room temperature for 20 min. The suspension was filtered and the solid was dried in vacuo to afford the title compound (96.0 g, 84 %) as a yellow solid. MS: 345.9 ([{⁷⁹Br, ³⁵Cl}M+H]⁺), 347.8 ([{⁸¹Br, ³⁵Cl or ⁷⁹Br, ³⁷Cl }M+H]⁺), ESI pos.

### e) 7-bromo-6-chloro-5-(2,6-difluorophenyl)-1,3-dihydro-1,4-benzodiazepin-2-one

To a solution of (6-amino-3-bromo-2-chloro-phenyl)-(2,6-difluorophenyl)methanone (25.0 g, 72.1 mmol) in pyridine (625 mL) was added ethyl 2-aminoacetate hydrochloride (70.5 g, 505 mmol). The reaction mixture was stirred at 135 °C for 36 h. The reaction mixture was concentrated in vacuo to remove pyridine. The residue was diluted with ethyl acetate (2000 mL) and washed with aqueous HCl (1.0 M, 3 × 1500 mL), water (2000 mL) and brine (2 × 1000 mL), dried (Na₂SO₄), filtered and concentrated in vacuo. The crude product was purified by flash column chromatography (silica, petroleum ether / ethyl acetate 10:1 to 2:1) to afford the title compound (10.1 g, 12 %) as an off-white solid. MS: 385.0 ([{⁷⁹Br, ³⁵Cl}M+H]⁺), ESI pos.

### f) 6-chloro-5-(2,6-difluorophenyl)-7-methyl-1,3-dihydro-1,4-benzodiazepin-2-one

A microwave tube was charged with 7-bromo-6-chloro-5-(2,6-difluorophenyl)-1,3-dihydro-1,4-benzodiazepin-2-one (450 mg, 1.17 mmol), trimethylboroxine (205 mg, 228 µL, 1.63 mmol), potassium carbonate (242 mg, 1.75 mmol) and tetrakis(triphenylphosphine)palladium (0) (67.4 mg, 58.4 µmol). Degassed 1,4-dioxane (8.1 mL) and H₂O (2.7 ml) were added then the vial was capped. The suspension was reacted in microwave at 130 °C for 30 min to give complete conversion. The mixture was evaporated, treated with sat. aq. NaHCO₃ (20 mL) and extracted with EtOAc (2 × 20 mL). The organic layers were dried (Na₂SO₄), filtered and solvents were evaporated. The residue was purified by flash column chromatography (silica, 40 g, CH₂Cl₂/EtOAc in heptane 10% to 40% to 70%) to give the title compound (344 mg, 92%) as light yellow solid. MS (ESI): 321.1 ([M+H]⁺).

### g) 6-chloro-5-(2,6-difluorophenyl)-7-methyl-1-(tritritiomethyl)-3H-1,4-benzodiazepin-2-one

To a solution of [³H]methyl nosylate (1.85 GBq, 50 mCi, 0.61 µmol) in THF (200 µL) were added the *N*-desmethyl precursor 6-chloro-5-(2,6-difluorophenyl)-7-methyl-1,3-dihydro-1,4-benzodiazepin-2-one (0.43 mg, 1.34 µmol) dissolved in THF (200 µL) and 10 equivalents of sodium *tert*-butylate (0.5 M in THF, 13.4 µmol). After stirring for 4 h at room temperature the reaction mixture was treated with H₂O, evaporated, and the crude product was purified by HPLC (X-Terra Prep RP-18, 10 x 150 mm, MeCN/H₂O (containing 5% of MeCN) 40:60, 4 ml/min, 230 nm). The pure tritium-labeled compound was isolated by solid phase extraction (Sep-Pak Plus C18) and eluted from the cartridge as ethanolic solution to yield 1.6 GBq (43.2 mCi) of the target compound in > 99% radiochemical purity and a specific activity of 2.49 TBq/mmol (67.3 Ci/mmol) as determined by mass spectrometry (MS). The identity of the labeled compound was confirmed by HPLC (by co-injecting the unlabeled reference standard) and by MS.

MS: m/z = 335 [M(H)+H]⁺ (16%), 337 [M(³H)+H]⁺ (0%), 339 [M(³H₂)+H]⁺ (16%), 341 [M(³H₃)+H]⁺ (68%).

### Membrane preparation and binding assay for γ2-containing GABA_{A} subtypes

The affinity of compounds at GABA_{A} γ2 subunit-containing receptors was measured by competition for [³H]Flumazenil (81.1 Ci/mmol; Roche) binding to HEK293F cells expressing human (transiently transfected) receptors of composition α1β3γ2.

Harvested pellets from HEK293F cells expressing the different GABA_{A} γ2 receptor subtypes were resuspended in Mannitol Buffer pH 7,2 -7,4 and processed as described above for the cells expressing the GABA_{A} γ1 subunit-containing receptors.

Radioligand binding assays were carried out in a volume of 200 µL (96-well plates) which contained 100 µL of cell membranes, [³H]Flumazenil at a concentration of 1 nM and the test compound in the range of [0.1 · 10⁻³-10]× 10⁻⁶ M. Nonspecific binding was defined by 10⁻⁵ M Diazepam and typically represented less than 5% of the total binding. Assays were incubated to equilibrium for 1 hour at 4 °C and harvested onto GF/C uni-filters (Packard) by filtration using a Packard harvester and washing with ice-cold wash buffer (50 mM Tris; pH 7.5). After anhydrousing, filter-retained radioactivity was detected by liquid scintillation counting. Kᵢ values were calculated using Excel-Fit (Microsoft) and are the means of two determinations.

The compounds of the accompanying examples were tested in the above described assay, and the preferred compounds were found to possess large Kᵢ value for displacement of [³H]Flumazenil from the α1β3γ2 subtype of the human GABA_{A} receptor of 100 nM or above. Most preferred are compounds with a Kᵢ α1β3γ2 (nM) > 300. In a preferred embodiment the compounds of the invention are binding selectively for the γ1 subunit-containing GABA_{A} receptors relative to γ2 subunit-containing GABA_{A} receptors. In particular, compounds of the present invention have γ2/γ1 selectivity ratio defined as "Kᵢ α1β3γ2 (nM) / Kᵢ α2β2γ1 (nM)" above 10-fold, or LogSel defined as "Log[Kᵢ α1β3γ2 (nM) / Kᵢ α2β2γ1 (nM)]" above 1. Representative test results, obtained by the above described assay measuring binding affinity to HEK293 cells expressing human (h) receptors, are shown in the Table 1 below.

**Table 1**

| **Example** | **Ki h-GABA_{A} α5β2γ1 (nM)** | **Ki h-GABA_{A} α2β2γ1 (nM)** | **Ki h-GABA_{A} α1β2γ1 (nM)** | **Ki h-GABA_{A} α1β3γ2 (nM)** | **γ2/γ1 Selectivity** | **LogSel** |
|---|---|---|---|---|---|---|
| | | | | | **Ratio** | |
| **1** | 2.4 | 31.7 | ND | 1086 | 34 | 1.53 |
| **2** | 12.6 | 142.8 | ND | 3294 | 23 | 1.36 |
| **3** | 4.7 | 51.6 | ND | 1792 | 35 | 1.54 |
| **4** | 4.9 | 64.3 | 51.6 | 7270 | 113 | 2.05 |
| **5** | 1.7 | 40.0 | ND | 4737 | 118 | 2.07 |
| **6** | 11.6 | 45.2 | ND | 10248 | 227 | 2.36 |
| **7** | 3.5 | 20.2 | ND | 5099 | 253 | 2.40 |
| **8** | 2.5 | 14.6 | 48.8 | 3723 | 255 | 2.41 |
| **9** | 7.1 | 19.9 | 45.8 | 3386 | 170 | 2.23 |
| **10** | 3.0 | 14.3 | 80.5 | 4611 | 321 | 2.51 |
| **11** | 4.6 | 9.6 | 45.8 | 7894 | 818 | 2.91 |
| **12** | 5.2 | 69.2 | ND | 12305 | 178 | 2.25 |
| **13** | 3.1 | 32.0 | ND | 5301 | 166 | 2.22 |
| **14** | 3.7 | 46.0 | 91.8 | 8759 | 190 | 2.28 |
| **15** | 2.6 | 34.5 | 114.4 | 9473 | 275 | 2.44 |
| **16** | 4.1 | 39.8 | 156.7 | 9473 | 238 | 2.38 |
| **17** | 3.8 | 22.6 | 34.4 | 4871 | 215 | 2.33 |
| **18** | 5.9 | 54.5 | ND | 4174 | 77 | 1.88 |
| **19** | 3.8 | 21.2 | 48.9 | 5105 | 241 | 2.38 |
| **20** | 4.9 | 11.8 | ND | 1087 | 92 | 1.96 |
| **21** | 10.7 | 72.3 | ND | 11292 | 156 | 2.19 |
| **22** | 6.4 | 43.2 | ND | 10575 | 245 | 2.39 |
| **23** | 9.7 | 101.3 | ND | 14543 | 144 | 2.16 |
| **24** | 2.8 | 72.6 | ND | 8376 | 115 | 2.06 |

### Functional expression of GABA_{A} receptors:

### Xenopus oocytes preparation

Xenopus laevis oocytes at maturation stages V-VI were used for the expression of cloned mRNA encoding GABA_{A} receptor subunits. Oocytes ready for RNA micro-injection were bought from Ecocyte, Castrop-Rauxel, Germany and stored in modified Barth's medium (composition in mM: NaCl 88, KCl 1, NaHCO₃ 2.4, HEPES 10, MgSO₄ 0.82, CaNO₃ 0.33, CaCl₂ 0.33, pH = 7.5) at 20 °C until the experiment.

### Xenopus oocytes microinjection

Oocytes were plated in 96-well plates for microinjection using the Roboinject automated instrument (MultiChannelSystems, Reutlingen, Germany). Approximately 50 nL of an aqueous solution containing the RNA transcripts for the subunits of the desired GABA_{A} receptor subtype was injected into each oocyte. RNA concentrations ranged between 20 and 200 pg/µL/subunit and were adjusted in pilot experiments to obtain GABA responses of a suitable size and a maximal effect of Flunitrazepam, Triazolam and Midazolam, reference benzodiazepine positive allosteric modulators (PAM) at the GABA_{A} receptor benzodiazepine (BZD) binding site. Oocytes were kept in modified Barth's medium (composition in mM: NaCl 88, KCl 1, NaHCO₃ 4, HEPES 10, MgSO₄ 0.82, CaNO₃ 0.33, CaCl₂ 0.33, pH = 7.5) at 20°C until the experiment.

### Electrophysiology

Electrophysiological experiments were performed using the Roboocyte instrument (MultiChannelSystems, Reutlingen, Germany) on days 3 to 5 after the micro-injection of mRNA. During the experiment the oocytes were constantly superfused by a solution containing (in mM) NaCl 90, KCl 1, HEPES 5, MgCl₂ 1, CaCl₂ 1 (pH 7.4). Oocytes were impaled by two glass microelectrodes (resistance: 0.5-0.8 MΩ) which were filled with a solution containing KCl 1M + K-acetate 1.5 M and voltage-clamped to -80 mV. The recordings were performed at room temperature using the Roboocyte two-electrode voltage clamp system (Multichannelsystem). After an initial equilibration period of 1.5 min GABA was added for 1.5 min at a concentration evoking approximately 20% of a maximal current response (EC₂₀). After another rest interval of 2.5 min GABA was again added evoking a response of similar amplitude and shape. 0.5 min after the onset of this second GABA application the test compound, at a concentration corresponding to approximatively 30-fold its Kᵢ α2β2γ1, was added while GABA was still present. Current traces were recorded at a digitization rate of 10 Hz during and shortly before and after the GABA application.

Each compound and concentration was tested on at least 3 oocytes. Different oocytes were used for different compound concentrations. The reference PAMs, Flunitrazepam, Triazolam and Midazolam, potentiated the GABA-induced current in α2β2γ1 GABA_{A} receptor subtype expressing oocytes by approximatively 60%.

### Data analysis

For the analysis, the digitized current traces of the first and second GABA response were superimposed and, if necessary, rescaled to equal maximal amplitudes. The ratio between the two responses during the time interval of test compound application was calculated point by point. The extremum of the resulting "ratio trace" was taken as the efficacy ("Fold increase") of the compound expressed as "% modulation of GABA EC₂₀" (100* (Fold increase-1)).

The results are shown in Table 2.

**Table 2**

| **Example** | **Ki h-GABA_{A} α2β2γ1 (nM)** | **Fold increase h-GABA-A α2β2γ1 oocyte @ 30-fold Ki** | **Efficacy (GABA)%** |
|---|---|---|---|
| **1** | 31.7 | 1.55 | 55 |
| **2** | 142.8 | 2.62 | 162 |
| **3** | 51.6 | 1.66 | 66 |
| **4** | 64.3 | 2.16 | 116 |
| **5** | 40.0 | 1.84 | 84 |
| **6** | 45.2 | 1.91 | 91 |
| **7** | 20.2 | 1.85 | 85 |
| **8** | 14.6 | 2.14 | 114 |
| **9** | 19.9 | 1.87 | 87 |
| **10** | 14.3 | 1.82 | 82 |
| **11** | 9.6 | 2.14 | 114 |
| **12** | 69.2 | 2.43 | 143 |
| **13** | 32.0 | 2.73 | 173 |
| **14** | 39.2 | 2.58 | 158 |
| **15** | 36.5 | 2.46 | 146 |
| **16** | 35.3 | 2.66 | 166 |
| **17** | 21.0 | 1.91 | 91 |
| **18** | 131.3 | ND | -- |
| **19** | 23.2 | 2.70 | 170 |
| **20** | 15.1 | 2.08 | 108 |
| **21** | 72.3 | 2.75 | 175 |
| **22** | 43.2 | 2.72 | 172 |
| **23** | 101.3 | ND | -- |
| **24** | 72.6 | ND | -- |

### Reference compounds

Benzodiazepines reference compounds (classical marketed benzodiazepines) and reference thieno-diazepines listed below were tested for their affinity towards the GABA_{A} receptor α1β2γ1 and α2β2γ1subtypes as well as in the GABA_{A} receptor α1β3γ2 subtype. The results are shown in Table 3.

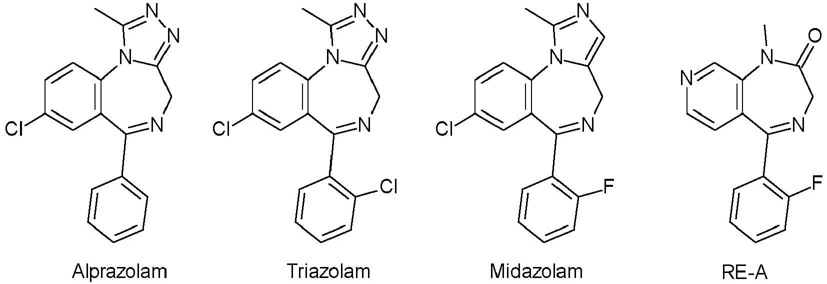

**Table 3**

| **Example** | **Ki h-GABA_{A} α1β2γ1 (nM)** | **Ki h-GABA_{A} α2β2γ1 (nM)** | **Ki h-GABA_{A} α1β3γ2 (nM)** | **γ2/γ1 Selectivity** | **LogSel** |
|---|---|---|---|---|---|
| | | | | **Ratio** | |
| **Alprazolam** | 5923 | 3945 | 19.6 | 0.0050 | -2.3 |
| **Triazolam** | 44.2 | 46.2 | 1.5 | 0.032 | -1.5 |
| **Midazolam** | 1153.2 | 737.7 | 5.0 | 0.0068 | -2.2 |
| **RE-A** | ND | 10191 | 178.9 | 0.018 | -1.8 |
| **Example 4** | 51.6 | 64.3 | 7270 | 113 | 2.1 |
| **Example 10** | 80.5 | 14.3 | 4611 | 321 | 2.5 |

RE-A is disclosed in Drug Design and Discovery (1993), 10(1), 45-55 (Synthesis and anticonvulsant activity of 1,3-dihydro-5-phenyl-2H-pyrido[3,4-e]-1,4-diazepin-2-ones).

### Preparation of pharmaceutical compositions comprising compounds of the invention

Tablets comprising compounds of formula (I) are manufactured as follows:

| **Ingredient** | **mg/tablet** | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

Capsules comprising compounds of formula (I) are manufactured as follows:

| **Ingredient** | **mg/capsule** | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

A compound of formula I lactose and corn starch are firstly mixed in a mixer and then in a comminuting machine. The mixture is returned to the mixer; the talc is added thereto and mixed thoapproximatively. The mixture is filled by machine into suitable capsules, e.g. hard gelatin capsules.

Injection solutions comprising compounds of formula (I) are manufactured as follows:

| **Ingredient** | **mg/injection solution.** |
|---|---|
| Compound of formula I | 3 |
| Polyethylene Glycol 400 | 150 |
| acetic acid | q.s. ad pH 5.0 |
| water for injection solutions | ad 1.0 ml |

## Claims

1. A compound of formula (I) 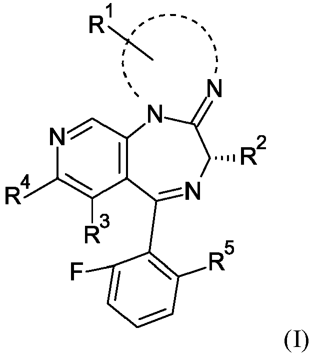 or a pharmaceutically acceptable salt thereof, wherein: 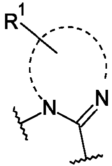 is selected from: 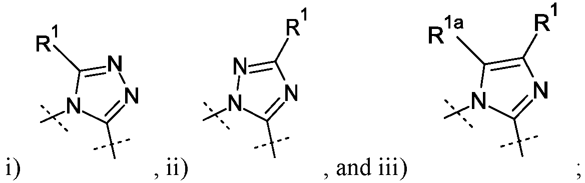
R¹ is selected from hydrogen, C₁-C₆-alkyl, hydroxy-C₁-C₆-alkyl-NH-C(O)-, and a group 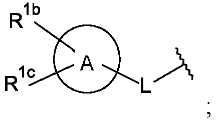 and R^{1a} is hydrogen; or
R¹ and R^{1a}, taken together with the carbon atoms to which they are attached, form a C₃-C₁₀-cycloalkyl;
R^{1b} is selected from hydrogen, halogen, hydroxy, oxo, C₁-C₆-alkyl, and C₁-C₆-alkoxy;
R^{1c} is selected from hydrogen, hydroxy, and oxo;
R² is C₁-C₆-alkyl;
R³ is chloro or bromo;
R⁴ is selected from halogen, C₁-C₆-alkyl, halo-C₁-C₆-alkyl, and C₃-C₁₀-cycloalkyl;
R⁵ is halogen;
L is selected from a covalent bond, carbonyl, -C(O)NH-,-NHC(O)-, and -CH₂NHC(O)-; and
A is selected from 3-14-membered heterocycloalkyl and C₃-C₁₀-cycloalkyl.

2. The compound of formula (I) according to claim 1, or a pharmaceutically acceptable salt thereof, wherein
R¹ is selected from C₁-C₆-alkyl, hydroxy-C₁-C₆-alkyl-NH-C(O)-, and a group 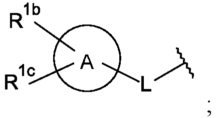 and R^{1a} is hydrogen; or
R¹ and R^{1a}, taken together with the carbon atoms to which they are attached, form a C₃-C₁₀-cycloalkyl; and
R^{1b}, R^{1c}, A, and L are as defined in claim 1.

3. The compound of formula (I) according to claim 2, or a pharmaceutically acceptable salt thereof, wherein
R¹ is selected from C₁-C₆-alkyl, hydroxy-C₁-C₆-alkyl-NH-C(O)-, and a group 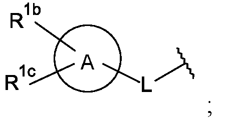
R^{1b} is C₁-C₆-alkyl;
R^{1c} is hydroxy;
L is carbonyl; and
A is a 3-14-membered heterocycle.

4. The compound of formula (I) according to claim 3, or a pharmaceutically acceptable salt thereof, wherein
R¹ is selected from 2-hydroxyethyl-NH-C(O)-, 2-hydroxypropyl-NH-C(O)-, methyl, and a group ; 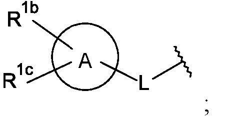
R^{1b} is methyl;
R^{1c} is hydroxy;
L is carbonyl; and
A is a azetidinyl.

5. The compound of formula (I) according to any one of claims 1 to 4, or a pharmaceutically acceptable salt thereof, wherein R² is methyl.

6. The compound of formula (I) according to any one of claims 1 to 5, or a pharmaceutically acceptable salt thereof, wherein R³ is chloro.

7. The compound of formula (I) according to any one of claims 1 to 6, or a pharmaceutically acceptable salt thereof, wherein R⁴ is halo-C₁-C₆-alkyl.

8. The compound of formula (I) according to claim 7, or a pharmaceutically acceptable salt thereof, wherein R⁴ is CF₃.

9. The compound of formula (I) according to any one of claims 1 to 8, or a pharmaceutically acceptable salt thereof, wherein R⁵ is fluoro.

10. The compound of formula (I) according to claim 1, or a pharmaceutically acceptable salt thereof, wherein:
R¹ is selected from C₁-C₆-alkyl, hydroxy-C₁-C₆-alkyl-NH-C(O)-, and a group 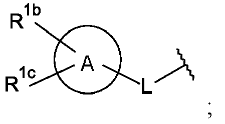
R^{1b} is C₁-C₆-alkyl;
R^{1c} is hydroxy;
R² is C₁-C₆-alkyl;
R³ is chloro;
R⁴ is halo-C₁-C₆-alkyl;
R⁵ is halogen;
L is carbonyl; and
A is a 3-14-membered heterocycle.

11. The compound of formula (I) according to claim 10, or a pharmaceutically acceptable salt thereof, wherein:
R¹ is selected from methyl, 2-hydroxyethyl-NH-C(O)-, 2-hydroxypropyl-NH-C(O)-, and a group 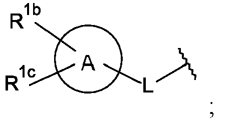
R^{1b} is methyl;
R^{1c} is hydroxy;
R² is methyl;
R³ is chloro;
R⁴ is CF₃;
R⁵ is fluoro;
L is carbonyl; and
A is azetidinyl.

12. The compound of formula (I) according to claim 1, or a pharmaceutically acceptable salt thereof, wherein said compound of formula (I) is selected from:
(7*S*)-11*,*12-dichloro-9-(2,6-difluorophenyl)-3,7-dimethyl-2,4,5,8,13-pentazatricyclo[8.4.0.02,6]tetradeca-1(10),3,5,8,11,13-hexaene;
(7*S*)-11-chloro-12-cyclopropyl-9-(2,6-difluorophenyl)-3,7-dimethyl-2,4,5,8,13-pentazatricyclo[8.4.0.02,6]tetradeca-1(10),3,5,8,11,13-hexaene;
(7*s*)-11-chloro-9-(2,6-difluorophenyl)-3,7,12-trimethyl-2,4,5,8,13-pentazatricyclo[8.4.0.02,6]tetradeca-1(10),3,5,8,11,13-hexaene;
(7*S*)-11-chloro-9-(2,6-difluorophenyl)-3,7-dimethyl-12-(trifluoromethyl)-2,4,5,8,13-pentazatricyclo[8.4.0.02,6]tetradeca-1(10),3,5,8,11,13-hexaene;
(7*S*)-11-chloro-9-(2,6-difluorophenyl)-7-methyl-12-(trifluoromethyl)-2,3,5,8,13 - pentazatricyclo[8.4.0.02,6]tetradeca-1(10),3,5,8,11,13-hexaene;
azetidin-1-yl-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-fluoroazetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxyazetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-methoxyazetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanone;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(1,1-dioxo-1,4-thiazinan-4-yl)methanone;
*N*-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]oxetane-3-carboxamide;
1-[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]pyrrolidin-2-one;
(7*S*)-11-chloro-9-(2,6-difluorophenyl)-*N*-[(2*S*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-(2-hydroxyethyl)-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-[(2*R*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2-chloro-6-fluoro-phenyl)-3,7-dimethyl-12-(trifluoromethyl)-2,4,5,8,13-pentazatricyclo[8.4.0.02,6]tetradeca-1(10),3,5,8,11,13-hexaene;
(7*S*)-11-chloro-9-(2,6-difluorophenyl)-4,7-dimethyl-12-(trifluoromethyl)-2,5,8,13-tetrazatricyclo[8.4.0.02,6]tetradeca-1(10),3,5,8,11,13-hexaene;
(7*S*)-11-chloro-9-(2,6-difluorophenyl)-*N*-(2-hydroxyethyl)-7-methyl-12-(trifluoromethyl)-2,5,8,13-tetrazatricyclo[8.4.0.02,6]tetradeca-1(10),3,5,8,11,13-hexaene-4-carboxamide;
(10*S*)-6-chloro-8-(2,6-difluorophenyl)-10-methyl-5-(trifluoromethyl)-1,4,9,12-tetrazatetracyclo[9.6.0.02,7.013,17]heptadeca-2(7),3,5,8,11,13(17)-hexaene;
(7*S*)-11-chloro-9-(2,6-difluorophenyl)-*N*-(2-hydroxy-2-methyl-propyl)-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-[(1-hydroxycyclopropyl)methyl]-7-methyl-12-(trifluoromethyl)-2,3,5,8,13-pentazatricyclo[8.4.0.02,6]tetradeca-1(10),3,5,8,11,13-hexaene-4-carboxamide;
(7S)-11-chloro-9-(2,6-difluorophenyl)-N-cis-(3-hydroxycyclobutyl)-7-methyl-12-(trifluoromethyl)-2,3,5,8,13-pentazatricyclo[8.4.0.02,6]tetradeca-1(10),3,5,8,11,13-hexaene-4-carboxamide; and
(7S)-11-chloro-9-(2,6-difluorophenyl)-N-trans-(3-hydroxycyclobutyl)-7-methyl-12-(trifluoromethyl)-2,3,5,8,13-pentazatricyclo[8.4.0.02,6]tetradeca-1(10),3,5,8,11,13-hexaene-4-carboxamide.

13. The compound of formula (I) according to claim 12, or a pharmaceutically acceptable salt thereof, wherein said compound of formula (I) is selected from:
(7*S*)-11-chloro-9-(2,6-difluorophenyl)-3,7-dimethyl-12-(trifluoromethyl)-2,4,5,8,13-pentazatricyclo[8.4.0.02,6]tetradeca-1(10),3,5,8,11,13-hexaene;
[(7*S*)-11-chloro-9-(2,6-difluorophenyl)-7-methyl-12-(trifluoromethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanone;
(7*S*)-11-chloro-9-(2,6-difluorophenyl)-*N*-[(2*S*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-(2-hydroxyethyl)-7-methyl-12-(trifluoromethyl)-2,3,5,8,13-pentazatricyclo[8.4.0.02,6]tetradeca-1(10),3,5,8,11,13-hexaene-4-carboxamide;
(7*S*)-11-chloro-9-(2,6-difluorophenyl)-*N*-[(2*R*)-2-hydroxypropyl]-7-methyl-12-(trifluoromethyl)-2,3,5,8,13-pentazatricyclo[8.4.0.02,6]tetradeca-1(10),3,5,8,11,13-hexaene-4-carboxamide; and
(7*S*)-11-chloro-9-(2,6-difluorophenyl)-*N*-(2-hydroxyethyl)-7-methyl-12-(trifluoromethyl)-2,5,8,13-tetrazatricyclo[8.4.0.02,6]tetradeca-1(10),3,5,8,11,13-hexaene-4-carboxamide.

14. A compound of formula (I) according to any one of claims 1 to 13, or a pharmaceutically acceptable salt thereof, for use as therapeutically active substance.

15. A pharmaceutical composition comprising a compound of formula (I) according to any one of claims 1 to 13, or a pharmaceutically acceptable salt thereof, and a therapeutically inert carrier.

16. A compound of formula (I) according to any of claims 1 to 13, or a pharmaceutically acceptable salt thereof, or a pharmaceutical composition according to claim 15, for use in treating or preventing acute neurological disorders, chronic neurological disorders and/or cognitive disorders in a subject.

## Patentansprüche

1. Verbindung der Formel (I) 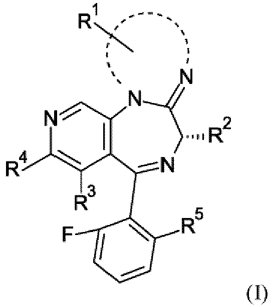 oder ein pharmazeutisch verträgliches Salz davon, wobei: 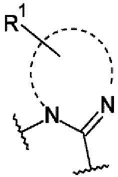 ist ausgewählt aus: 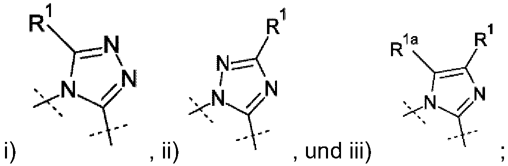
R¹ ist ausgewählt aus Wasserstoff, C₁-C₆-Alkyl, Hydroxy-C₁-C₆-Alkyl-NH-C(O)- und einer Gruppe 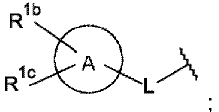 und R^{1a} ist Wasserstoff; oder
R¹ und R^{1a} bilden zusammen mit den Kohlenstoffatomen, mit denen sie verknüpft sind, ein C₃-C₁₀-Cycloalkyl;
R^{1b} ist ausgewählt aus Wasserstoff, Halogen, Hydroxy, Oxo, C₁-C₆-Alkyl und C₁-C₆-Alkoxy;
R^{1c} ist ausgewählt aus Wasserstoff, Hydroxy und Oxo;
R² ist C₁-C₆-Alkyl;
**R³** ist Chloro oder Bromo;
**R⁴** ist ausgewählt aus Halogen, C₁-C₆-Alkyl, Halo-C₁-C₆-Alkyl und C₃-C₄₀-Cycloalkyl;
R⁵ ist Halogen;
L ist ausgewählt aus einer kovalenten Bindung, Carbonyl, -C(O)NH-, -NHC(O)- und - CH₂NHC(O)-; und
A ist ausgewählt aus 3-14-gliedrigem Heterocycloalkyl und C₃-C₁₀-Cycloalkyl.

2. Verbindung der Formel (I) nach Anspruch 1 oder ein pharmazeutisch verträgliches Salz davon, wobei
R¹ ist ausgewählt aus C₁-C₆-Alkyl, Hydroxy-C₁-C₆-Alkyl-NH-C(O)- und einer Gruppe 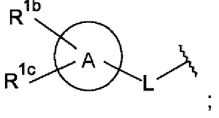 und R^{1a} ist Wasserstoff; oder
R¹ und R^{1a} bilden zusammen mit den Kohlenstoffatomen, mit denen sie verknüpft sind, ein C₃-C₁₀-Cycloalkyl; und
R^{1b}, R^{1c}, A und L sind wie in Anspruch 1 definiert.

3. Verbindung der Formel (I) nach Anspruch 2 oder ein pharmazeutisch verträgliches Salz davon, wobei
R¹ ist ausgewählt aus C₁-C₆-Alkyl, Hydroxy-C₁-C₆-Alkyl-NH-C(O)- und einer Gruppe 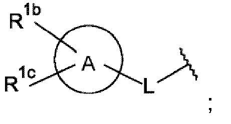
R^{1b} ist C₁-C₆-Alkyl;
R^{1c} ist Hydroxy;
L ist Carbonyl; und
A ist ein 3-14-gliedriger Heterozyklus.

4. Verbindung der Formel (I) nach Anspruch 3 oder ein pharmazeutisch verträgliches Salz davon, wobei
R¹ ist ausgewählt aus 2-Hydroxyethyl-NH-C(O)-, 2-Hydroxypropyl-NH-C(O)-, Methyl und einer Gruppe 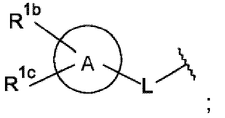
R^{1b} ist Methyl;
R^{1c} ist Hydroxy;
L ist Carbonyl; und
A ist ein Azetidinyl.

5. Verbindung der Formel (I) nach einem der Ansprüche 1 bis 4 oder ein pharmazeutisch verträgliches Salz davon, wobei R² Methyl ist.

6. Verbindung der Formel (I) nach einem der Ansprüche 1 bis 5 oder ein pharmazeutisch verträgliches Salz davon, wobei R³ Chloro ist.

7. Verbindung der Formel (I) nach einem der Ansprüche 1 bis 6 oder ein pharmazeutisch verträgliches Salz davon, wobei R⁴ Halo-C₁-C₆-Alkyl ist.

8. Verbindung der Formel (I) nach Anspruch 7 oder ein pharmazeutisch verträgliches Salz davon, wobei R⁴ CF₃ ist.

9. Verbindung der Formel (I) nach einem der Ansprüche 1 bis 8 oder ein pharmazeutisch verträgliches Salz davon, wobei R⁵ Fluoro ist.

10. Verbindung der Formel (I) nach Anspruch 1 oder ein pharmazeutisch verträgliches Salz davon, wobei:
R¹ ist ausgewählt aus C₁-C₆-Alkyl, Hydroxy-C₁-C₆-Alkyl-NH-C(O)- und einer Gruppe 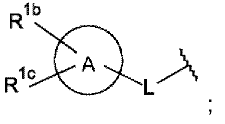
R^{1b} ist C₁-C₆-Alkyl;
R^{1c} ist Hydroxy;
R² ist C₁-C₆-Alkyl;
**R³** ist Chloro;
**R⁴** ist Halo-C₁-C₆-Alkyl;
R⁵ ist Halogen;
L ist Carbonyl; und
A ist ein 3-14-gliedriger Heterozyklus.

11. Verbindung der Formel (I) nach Anspruch 10 oder ein pharmazeutisch verträgliches Salz davon, wobei:
R¹ ist ausgewählt aus Methyl, 2-Hydroxyethyl-NH-C(O)-, 2-Hydroxypropyl-NH-C(O)- und einer Gruppe 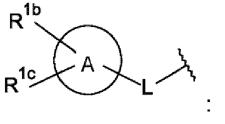
R^{1b} ist Methyl;
R^{1c} ist Hydroxy;
R² ist Methyl;
R³ ist Chloro;
R⁴ ist CF₃;
R⁵ ist Fluoro;
L ist Carbonyl; und
A ist Azetidinyl.

12. Verbindung der Formel (I) nach Anspruch 1 oder ein pharmazeutisch verträgliches Salz davon, wobei die Verbindung der Formel (I) ausgewählt ist aus:
(7*S*)-11,12-Dichlor-9-(2,6-difluorphenyl)-3,7-dimethyl-2,4,5,8,13-pentazatricyclo[8.4.0.02,6]tetradeca-1(10),3,5,8,11,13-hexaen;
(7*S*)-11-Chlor-12-cyclopropyl-9-(2,6-difluorphenyl)-3,7-dimethyl-2,4,5,8,13-pentazatricyclo[8.4.0.02,6]tetradeca-1(10),3,5,8,11,13-hexaen;
(7*S*)-11-Chlor-9-(2,6-difluorphenyl)-3,7,12-trimethyl-2,4,5,8,13-pentazatricyclo[8.4.0.02,6]tetradeca-1(10),3,5,8,11,13-hexaen;
(7*S*)-11-Chlor-9-(2,6-difluorphenyl)-3,7-dimethyl-12-(trifluormethyl)-2,4,5,8,13-pentazatricyclo[8.4.0.02,6]tetradeca-1(10),3,5,8,11,13-hexaen;
(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen;
Azetidin-1-yl-[(7*S*)-11-chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]methanon;
[(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]-(3-fluorazetidin-1-yl)methanon;
[(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]-(3-hydroxyazetidin-1-yl)methanon;
[(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]-(3-methoxyazetidin-1-yl)methanon;
[(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanon;
[(7S)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]-(1,1-dioxo-1,4-thiazinan-4-yl)methanon;
*N*-[(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]oxetan-3-carboxamid;
1-[(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]pyrrolidin-2-on;
(7*S*)-11-Chlor-9-(2,6-difluorphenyl)-*N*-[(2*S*)-2-hydroxypropyl]-7-methyl-12-(trifluormethyl)-2,3,5,8,13-pentazatricyclo[8.4.0.02,6]tetradeca-1(10),3,5,8,11,13-hexaen-4-carboxamid;
(7*S*)-11-Chlor-9-(2,6-difluorphenyl)-*N*-(2-hydroxyethyl)-7-methyl-12-(trifluormethyl)-2,3,5,8,13-pentazatricyclo[8.4.0.02,6]tetradeca-1(10),3,5,8,11,13-hexaen-4-carboxamid;
(7*S*)-11-Chlor-9-(2,6-difluorphenyl)-*N*-[(2*R*)-2-hydroxypropyl]-7-methyl-12-(trifluormethyl)-2,3,5,8,13-pentazatricyclo[8.4.0.02,6]tetradeca-1(10),3,5,8,11,13-hexaen-4-carboxamid;
(7*S*)-11-Chlor-9-(2-chlor-6-fluor-phenyl)-3,7-dimethyl-12-(trifluormethyl)-2,4,5,8,13-pentazatricyclo[8.4.0.02,6]tetradeca-1(10),3,5,8,11,13-hexaen;
(7*S*)-11-Chlor-9-(2,6-difluorphenyl)-4,7-dimethyl-12-(trifluormethyl)-2,5,8,13-tetrazatricyclo[8.4.0.02,6]tetradeca-1(10),3,5,8,11,13-hexaen;
(7*S*)-11-Chlor-9-(2,6-difluorphenyl)-*N*-(2-hydroxyethyl)-7-methyl-12-(trifluormethyl)-2,5,8,13-tetrazatricyclo[8.4.0.02,6]tetradeca-1(10),3,5,8,11,13-hexaen-4-carboxamid;
(10*S*)-6-Chlor-8-(2,6-difluorphenyl)-10-methyl-5-(trifluormethyl)-1,4,9,12-tetrazatetracyclo[9.6.0.02,7.013,17]heptadeca-2(7),3,5,8,11,13(17)-hexaen;
(7*S*)-11-Chlor-9-(2,6-difluorphenyl)-*N*-(2-hydroxy-2-methyl-propyl)-7-methyl-12-(trifluormethyl)-2,3,5,8,13-pentazatricyclo[8.4.0.02,6]tetradeca-1(10),3,5,8,11,13-hexaen-4-carboxamid;
(7*S*)-11-Chlor-9-(2,6-difluorphenyl)-*N*-[(1-hydroxycyclopropyl)methyl]-7-methyl-12-(trifluormethyl)-2,3,5,8,13-pentazatricyclo[8.4.0.02,6]tetradeca-1(10),3,5,8,11,13-hexaen-4-carboxamid;
(7*S*)-11-Chlor-9-(2,6-difluorphenyl)-*N*-*cis*-(3-hydroxycyclobutyl)-7-methyl-12-(trifluormethyl)-2,3,5,8,13-pentazatricyclo[8.4.0.02,6]tetradeca-1(10),3,5,8,11,13-hexaen-4-carboxamid; und
(7*S*)-11-Chlor-9-(2,6-difluorphenyl)-*N*-*trans*-(3-hydroxycyclobutyl)-7-methyl-12-(trifluormethyl)-2,3,5,8,13-pentazatricyclo[8.4.0.02,6]tetradeca-1(10),3,5,8,11,13-hexaen-4-carboxamid.

13. Verbindung der Formel (I) nach Anspruch 12 oder ein pharmazeutisch verträgliches Salz davon, wobei die Verbindung der Formel (I) ausgewählt ist aus:
(7*S*)-11-Chlor-9-(2,6-difluorphenyl)-3,7-dimethyl-12-(trifluormethyl)-2,4,5,8,13-pentazatricyclo[8.4.0.02,6]tetradeca-1(10),3,5,8,11,13-hexaen;
[(7*S*)-11-Chlor-9-(2,6-difluorphenyl)-7-methyl-12-(trifluormethyl)-2,3,5,8,13-pentazatricyclo[8.4.0.02,6]tetradeca-1(10),3,5,8,11,13-hexaen-4-yl]-(3-hydroxy-3-methyl-azetidin-1-yl)methanon;
(7*S*)-11-Chlor-9-(2,6-difluorphenyl)-*N*-[(2*S*)-2-hydroxypropyl]-7-methyl-12-(trifluormethyl)-2,3,5,8,13-pentazatricyclo[8.4.0.02,6]tetradeca-1(10),3,5,8,11,13-hexaen-4-carboxamid;
(7*S*)-11-Chlor-9-(2,6-difluorphenyl)-*N*-(2-hydroxyethyl)-7-methyl-12-(trifluormethyl)-2,3,5,8,13-pentazatricyclo[8.4.0.02,6]tetradeca-1(10),3,5,8,11,13-hexaen-4-carboxamid;
(7*S*)-11-Chlor-9-(2,6-difluorphenyl)-*N*-[(2*R*)-2-hydroxypropyl]-7-methyl-12-(trifluormethyl)-2,3,5,8,13-pentazatricyclo[8.4.0.02,6]tetradeca-1(10),3,5,8,11,13-hexaen-4-carboxamid; und
(7*S*)-11-Chlor-9-(2,6-difluorphenyl)-*N*-(2-hydroxyethyl)-7-methyl-12-(trifluormethyl)-2,5,8,13-tetrazatricyclo[8.4.0.02,6]tetradeca-1(10),3,5,8,11,13-hexaen-4-carboxamid.

14. Verbindung der Formel (I) nach einem der Ansprüche 1 bis 13 oder ein pharmazeutisch verträgliches Salz davon zur Verwendung als therapeutisch aktive Substanz.

15. Pharmazeutische Zusammensetzung, umfassend eine Verbindung der Formel (I) nach einem der Ansprüche 1 bis 13 oder ein pharmazeutisch verträgliches Salz davon und einen therapeutisch inerten Träger.

16. Verbindung der Formel (I) nach einem der Ansprüche 1 bis 13 oder ein pharmazeutisch verträgliches Salz davon oder pharmazeutische Zusammensetzung nach Anspruch 15 zur Verwendung beim Behandeln von oder Vorbeugen vor akuten neurologischen Störungen, chronischen neurologischen Störungen und/oder kognitiven Störungen bei einem Subjekt.

## Revendications

1. Composé de formule (I) 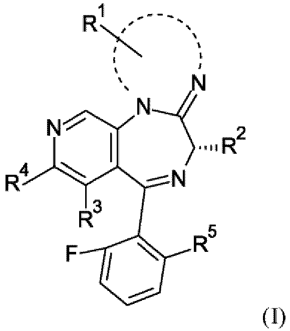 ou sel pharmaceutiquement acceptable de celui-ci, dans lequel : 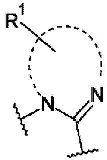 est choisi parmi : 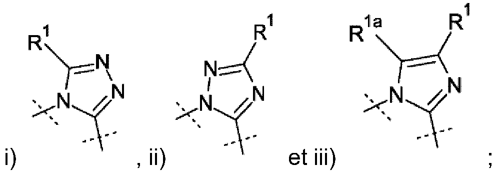
R¹ est choisi parmi hydrogène, alkyle en C₁-C₆, hydroxy-alkyle en C₁-C₆-NH-C(O)- et un groupe 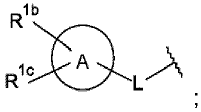 et R^{1a} est l'hydrogène ; ou
R¹ et R^{1a}, pris ensemble avec les atomes de carbone auxquels ils sont liés, forment un cycloalkyle en C₃-C₁₀ ;
R^{1b} est choisi parmi hydrogène, halogène, hydroxy, oxo, alkyle en C₁-C₆ et alcoxy en C₁-C₆ ;
R^{1c} est choisi parmi hydrogène, hydroxy et oxo ;
R² est un alkyle en C₁-C₆ ;
R³ est chloro ou bromo ;
R⁴ est choisi parmi halogène, alkyle en C₁-C₆, halo-alkyle en C₁-C₆ et cycloalkyle en C₃-C₁₀ ;
R⁵ est un halogène ;
L est choisi parmi une liaison covalente, carbonyle, -C(O)NH-, -NHC(O)- et -CH₂NHC(O)- ; et
A est choisi parmi hétérocycloalkyle à 3-14 chaînons et cycloalkyle en C₃-C₁₀.

2. Composé de formule (I) selon la revendication 1, ou sel pharmaceutiquement acceptable de celui-ci, dans lequel
R¹ est choisi parmi alkyle en C₁-C₆, hydroxy-alkyle en C₁-C₆-NH-C(O)- et un groupe 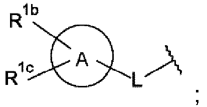 et R^{1a} est l'hydrogène ; ou
R¹ et R^{1a}, pris ensemble avec les atomes de carbone auxquels ils sont liés, forment un cycloalkyle en C₃-C₁₀ ; et
R^{1b}, R^{1c}, A et L sont tels que définis dans la revendication 1.

3. Composé de formule (I) selon la revendication 2, ou sel pharmaceutiquement acceptable de celui-ci, dans lequel
R¹ est choisi parmi alkyle en C₁-C₆, hydroxy-alkyle en C₁-C₆-NH-C(O)- et un groupe 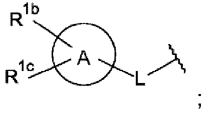
R^{1b} est un alkyle en C₁-C₆ ;
R^{1c} est hydroxy ;
L est carbonyle ; et
A est un hétérocycle à 3-14 chaînons.

4. Composé de formule (I) selon la revendication 3, ou sel pharmaceutiquement acceptable de celui-ci, dans lequel
R¹ est choisi parmi 2-hydroxyéthyle-NH-C(O)-, 2-hydroxypropyle-NH-C(O)-, méthyle et un groupe 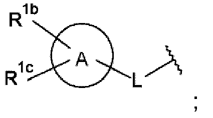
R^{1b} est méthyle ;
R^{1c} est hydroxy ;
L est carbonyle ; et
A est azétidinyle.

5. Composé de formule (I) selon l'une quelconque des revendications 1 à 4, ou sel pharmaceutiquement acceptable de celui-ci, dans lequel R² est méthyle.

6. Composé de formule (I) selon l'une quelconque des revendications 1 à 5, ou sel pharmaceutiquement acceptable de celui-ci, dans lequel R³ est chloro.

7. Composé de formule (I) selon l'une quelconque des revendications 1 à 6, ou sel pharmaceutiquement acceptable de celui-ci, dans lequel R⁴ est un halo-alkyle en C₁-C₆.

8. Composé de formule (I) selon la revendication 7, ou sel pharmaceutiquement acceptable de celui-ci, dans lequel R⁴ est CF₃.

9. Composé de formule (I) selon l'une quelconque des revendications 1 à 8, ou sel pharmaceutiquement acceptable de celui-ci, dans lequel R⁵ est fluoro.

10. Composé de formule (I) selon la revendication 1, ou sel pharmaceutiquement acceptable de celui-ci, dans lequel :
R¹ est choisi parmi alkyle en C₁-C₆, hydroxy-alkyle en C₁-C₆-NH-C(O)- et un groupe 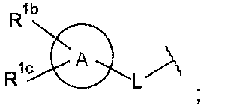
R^{1b} est un alkyle en C₁-C₆ ;
R^{1c} est hydroxy ;
R² est un alkyle en C₁-C₆ ;
R³ est chloro ;
R⁴ est un halo-alkyle en C₁-C₆ ;
R⁵ est un halogène ;
L est carbonyle ; et
A est un hétérocycle à 3-14 chaînons.

11. Composé de formule (I) selon la revendication 10, ou sel pharmaceutiquement acceptable de celui-ci, dans lequel :
R¹ est choisi parmi méthyle, 2-hydroxyéthyle-NH-C(O)-, 2-hydroxypropyle-NH-C(O)- et un groupe 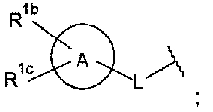
R^{1b} est méthyle ;
R^{1c} est hydroxy ;
R² est méthyle ;
R³ est chloro ;
R⁴ est CF3 ;
R⁵ est fluoro ;
L est carbonyle ; et
A est azétidinyle.

12. Composé de formule (I) selon la revendication 1, ou sel pharmaceutiquement acceptable de celui-ci, dans lequel ledit composé de formule (I) est choisi parmi :
(7*S*)-11,12-dichloro-9-(2,6-difluorophényl)-3,7-diméthyl-2,4,5,8,13-pentazatricyclo[8.4.0.02,6]tétradéca-1(10),3,5,8,11,13-hexaène ;
(7*S*)-11-chloro-12-cyclopropyl-9-(2,6-difluorophényl)-3,7-diméthyl-2,4,5,8,13-pentazatricyclo[8.4.0.02,6]tétradéca-1(10),3,5,8,11,13-hexaène ;
(7*S*)-11-chloro-9-(2,6-difluorophényl)-3,7,12-triméthyl-2,4,5,8,13-pentazatricyclo[8.4.0.02,6]tétradéca-1(10),3,5,8,11,13-hexaène ;
(7*S*)-11-chloro-9-(2,6-difluorophényl)-3,7-diméthyl-12-(trifluorométhyl)-2,4,5,8,13-pentazatricyclo[8.4.0.02,6]tétradéca-1(10),3,5,8,11,13-hexaène ;
(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaène ;
azétidin-1-yl-[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]méthanone ;
[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]-(3-fluoroazétidin-1-yl)méthanone ;
[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]-(3-hydroxyazétidin-1-yl)méthanone ;
[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]-(3-méthoxyazétidin-1-yl)méthanone ;
[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]-(3-hydroxy-3-méthyl-azétidin-1-yl)méthanone ;
[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]-(1,1-dioxo-1,4-thiazinan-4-yl)méthanone ;
*N*-[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]oxétane-3-carboxamide ;
1-[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]pyrrolidin-2-one ;
(7*S*)-11-chloro-9-(2,6-difluorophényl)-*N*-[(2*S*)-2-hydroxypropyl]-7-méthyl-12-(trifluorométhyl)-2,3,5,8,13-pentazatricyclo[8.4.0.02,6]tétradéca-1(10),3,5,8,11,13-hexaène-4-carboxamide ;
(7*S*)-11-chloro-9-(2,6-difluorophényl)-*N*-(2-hydroxyéthyl)-7-méthyl-12-(trifluorométhyl)-2,3,5,8,13-pentazatricyclo[8.4.0.02,6]tétradéca-1(10),3,5,8,11,13-hexaène-4-carboxamide ;
(7*S*)-11-chloro-9-(2,6-difluorophényl)-*N*-[(2*R*)-2-hydroxypropyl]-7-méthyl-12-(trifluorométhyl)-2,3,5,8,13-pentazatricyclo[8.4.0.02,6]tétradéca-1(10),3,5,8,11,13-hexaène-4-carboxamide ;
(7*S*)-11-chloro-9-(2-chloro-6-fluoro-phényl)-3,7-diméthyl-12-(trifluorométhyl)-2,4,5,8,13-pentazatricyclo[8.4.0.02,6]tétradéca-1(10),3,5,8,11,13-hexaène ;
(7*S*)-11-chloro-9-(2,6-difluorophényl)-4,7-diméthyl-12-(trifluorométhyl)-2,5,8,13-tétrazatricyclo[8.4.0.02,6]tétradéca-1(10),3,5,8,11,13-hexaène ;
(7*S*)-11-chloro-9-(2,6-difluorophényl)-*N*-(2-hydroxyéthyl)-7-méthyl-12-(trifluorométhyl)-2,5,8,13-tétrazatricyclo[8.4.0.02,6]tétradéca-1(10),3,5,8,11,13-hexaène-4-carboxamide ;
(10*S*)-6-chloro-8-(2,6-difluorophényl)-10-méthyl-5-(trifluorométhyl)-1,4,9,12-tétrazatétracyclo[9.6.0.02,7.013,17]heptadéca-2(7),3,5,8,11,13(17)-hexaène ;
(7*S*)-11-chloro-9-(2,6-difluorophényl)-*N*-(2-hydroxy-2-méthyl-propyl)-7-méthyl-12-(trifluorométhyl)-2,3,5,8,13-pentazatricyclo[8.4.0.02,6]tétradéca-1(10),3,5,8,11,13-hexaène-4-carboxamide ;
(7*S*)-11-chloro-9-(2,6-difluorophényl)-*N*-[(1-hydroxycyclopropyl)méthyl]-7-méthyl-12-(trifluorométhyl)-2,3,5,8,13-pentazatricyclo[8.4.0.02,6]tétradéca-1(10),3,5,8,11,13-hexaène-4-carboxamide ;
(7*S*)-11-chloro-9-(2,6-difluorophényl)-*N*-*cis*-(3-hydroxycyclobutyl)-7-méthyl-12-(trifluorométhyl)-2,3,5,8,13-pentazatricyclo[8.4.0.02,6]tétradéca-1(10),3,5,8,11,13-hexaène-4-carboxamide ; et
(7*S*)-11-chloro-9-(2,6-difluorophényl)-*N-trans*-(3-hydroxycyclobutyl)-7-méthyl-12-(trifluorométhyl)-2,3,5,8,13-pentazatricyclo[8.4.0.02,6]tétradéca-1(10),3,5,8,11,13-hexaène-4-carboxamide.

13. Composé de formule (I) selon la revendication 12, ou sel pharmaceutiquement acceptable de celui-ci, dans lequel ledit composé de formule (I) est choisi parmi :
(7*S*)-11-chloro-9-(2,6-difluorophényl)-3,7-diméthyl-12-(trifluorométhyl)-2,4,5,8,13-pentazatricyclo[8.4.0.02,6]tétradéca-1(10),3,5,8,11,13-hexaène ;
[(7*S*)-11-chloro-9-(2,6-difluorophényl)-7-méthyl-12-(trifluorométhyl)-2,3,5,8,13-pentazatricyclo[8.4.0.02,6]tétradéca-1(10),3,5,8,11,13-hexaén-4-yl]-(3-hydroxy-3-méthyl-azétidin-1-yl)méthanone ;
(7*S*)-11-chloro-9-(2,6-difluorophényl)-*N*-[(2*S*)-2-hydroxypropyl]-7-méthyl-12-(trifluorométhyl)-2,3,5,8,13-pentazatricyclo[8.4.0.02,6]tétradéca-1(10),3,5,8,11,13-hexaène-4-carboxamide ;
(7*S*)-11-chloro-9-(2,6-difluorophényl)-*N*-(2-hydroxyéthyl)-7-méthyl-12-(trifluorométhyl)-2,3,5,8,13-pentazatricyclo[8.4.0.02,6]tétradéca-1(10),3,5,8,11,13-hexaène-4-carboxamide ;
(7*S*)-11-chloro-9-(2,6-difluorophényl)-*N*-[(2*R*)-2-hydroxypropyl]-7-méthyl-12-(trifluorométhyl)-2,3,5,8,13-pentazatricyclo[8.4.0.02,6]tétradéca-1(10),3,5,8,11,13-hexaène-4-carboxamide ; et
(7*S*)-11-chloro-9-(2,6-difluorophényl)-*N*-(2-hydroxyéthyl)-7-méthyl-12-(trifluorométhyl)-2,5,8,13-tétrazatricyclo[8.4.0.02,6]tétradéca-1(10),3,5,8,11,13-hexaène-4-carboxamide.

14. Composé de formule (I) selon l'une quelconque des revendications 1 à 13, ou sel pharmaceutiquement acceptable de celui-ci, pour utilisation en tant que substance thérapeutiquement active.

15. Composition pharmaceutique comprenant un composé de formule (I) selon l'une quelconque des revendications 1 à 13, ou un sel pharmaceutiquement acceptable de celui-ci, et un véhicule thérapeutiquement inerte.

16. Composé de formule (I) selon l'une quelconque des revendications 1 à 13, ou sel pharmaceutiquement acceptable de celui-ci, ou composition pharmaceutique selon la revendication 15, pour utilisation dans le traitement ou la prévention de troubles neurologiques aigus, de troubles neurologiques chroniques et/ou de troubles cognitifs chez un sujet.
